# EUROPÄISCHE PATENTSCHRIFT

(11) **EP 3 878 394 B1**
(45) Veröffentlichungstag und Bekanntmachung des Hinweises auf die Patenterteilung: **02.04.2025**
(21) Anmeldenummer: 21159685.3
(22) Anmeldetag: 26.02.2021
(51) Int. Cl.: A61B 34/30, A61B 90/13, A61B 17/34

(54) **ANDOCKVORRICHTUNG UND CHIRURGISCHE HALTEVORRICHTUNG**
DOCKING DEVICE AND SURGICAL HOLDING DEVICE
DISPOSITIF D'ARRIMAGE ET DISPOSITIF CHIRURGICAL DE MAINTIEN

(30) Priorität: 12.03.2020 DE 102020106817
(43) Veröffentlichungstag der Anmeldung: 15.09.2021
(73) Patentinhaber: Karl Storz SE & Co. KG, 78532 Tuttlingen (DE)
(72) Erfinder: Zhang, Yaokun, Dr., 78532 Tuttlingen (DE); Klein, Kirsten, 78532 Tuttlingen (DE); Fallert, Johannes, Dr., 78532 Tuttlingen (DE)

(56) Entgegenhaltungen:
- EP-A2- 2 246 006
- WO-A1-2019/005921
- WO-A1-2019/096929
- WO-A1-2019/136062
- DE-A1- 102014 116 103
- JP-A- 2006 061 272
- SANAGOO ARASH: "Eine neuartige Roboterkinematik fur die laparoskopische Single-Port Chirurgie", DISSERTATION, 1 January 2015 (2015-01-01), pages 1 - 207, XP055790441, DOI: 10.18419/opus-4598

## Beschreibung

Die Erfindung betrifft eine Andockvorrichtung und ein Verfahren zum Verbinden eines chirurgischen Instruments mit einem Haltearm einer chirurgischen Haltevorrichtung, sowie eine chirurgische Haltevorrichtung mit einer solchen Andockvorrichtung.

Bei der Durchführung von chirurgischen Eingriffen werden in zunehmendem Maße Assistenzsysteme eingesetzt, um das medizinische Personal zu entlasten und Operationen zuverlässig und patientenschonend durchzuführen. Aus dem Stand der Technik ist es bekannt, dafür chirurgische Haltevorrichtungen bis hin zu Chirurgie-Robotern zum Halten und Positionieren von chirurgischen Instrumenten einzusetzen, mit denen über jeweils angeschlossenen Werkzeuge Handlungen am Körper des Patienten durchführbar sind. Dabei ist es üblich, dass das chirurgische Instrument bereits in dem Körper eines Patienten platziert ist, ehe es an die Haltevorrichtung angeschlossen wird.

Die EP 2 246 006 A2 beschreibt ein Operations-Assistenz-System, bei dem ein chirurgisches Instrument wie ein Endoskop an einen Arm eines Roboters angeschlossen wird, um es über dessen Kinematik bewegen zu können. Dazu ist ein Instrumententräger vorgesehen, der mit einem Kinematik-Arm über ein Drehgelenk lösbar verbunden wird und der weiter drehgelenkig mit einer Instrumentenhalterung verbunden ist. An diese Instrumentenhalterung wird das chirurgische Instrument angerastet. Eine für den folgenden Eingriff günstige Vorpositionierung findet nicht statt.

Die DE 10 2014 116 103 A1 offenbart ein Operation-Assistenz-System, das mehrere Roboterarme umfasst, die jeweils Schwenkachsen abbilden, die sich in unterschiedliche Richtungen erstrecken. Die Roboterarme sind miteinander an ihren Enden verbunden, wobei eine Tragsäule einen ersten Roboterarm trägt und an letzterem ein zweiter Roboterarm angelenkt ist. An einem freien Ende des zweiten Roboterarms ist ein Gelenkstück eingesetzt, das mit einem Instrumententräger verbunden ist, mit dem ein Endoskop gekoppelt werden kann. Eine Vorpositionierung des zu koppelnden Endoskops ist nicht vorgesehen.

JP2006061272A und WO2009005921 A1 offenbaren weiteren Stand der Technik.

Bei anderen Vorrichtungen wird das chirurgische Instrument, mit dem an einem Körper gearbeitet werden soll, oder der Trokar, in dem dieses geführt wird, direkt an den Haltearm bzw. Roboterkopf geklemmt. In beiden Fällen ist das Klemmen des Instruments mit starken Bewegungen des Instruments verbunden; beim Umpositionieren kann es zu Schäden an der Eintrittstelle des Körpers kommen, die umso schlimmer sein können, wenn der Pivotpunkt nicht präzise registriert ist.

Bei einem mikrochirurgischen, insbesondere laparoskopischen Eingriff, bei dem über einen in der Bauchdecke des Patienten gesetzten Trokar ein Zugang für das mikrochirurgische Instrument geschaffen wird, bildet der Trokarpunkt, d. h. der in der Bauchdecke liegende Mittelpunkt des Trokars, einen Pivotpunkt, um den das Instrument bewegbar ist und der selbst möglichst fix gehalten werden muss, um das Patientengewebe möglichst wenig zu strapazieren. Daher ist es insbesondere bei einer angetriebenen Kinematik notwendig, diesen Pivotpunkt im Steuerungssystem zu registrieren, damit beim Übertragen von Bewegungen auf das chirurgische Instrument, um dieses auf die gewünschte Weise im Körper des Patienten zu positionieren, die Bewegungen entsprechend um den fixen Pivotpunkt koordiniert werden können.

EP 2 254 735 B1 beschreibt dazu eine Vorab-Registrierung eines geplanten Trokarpunkts als Nullpunkt eines Koordinatensystems für ein Operations-Assistenz-System mittels eines Registertasters. Danach muss jedoch der Trokar entweder frei Hand oder über Schablonen oder Projizierungen am geplanten, registrierten Punkt platziert werden, was nicht präzise ist und auch nicht dem natürlichen Arbeitsablauf entspricht.

Teilweise kann der Pivotpunkt zunächst gesetzt und nachträglich registriert werden. Hierfür gibt es Trokare, deren Position über zwei senkrecht zueinander stehende Laserlinien aufgenommen wird, die auf die Bauchdecke projiziert werden, und die zum Trokarpunkt verfahren werden müssen, wobei die dritte Dimension erst durch am Trokar vorgesehene Tiefenmarkierungen erfassbar wird. Ferner ist es möglich, eine am Roboter montierte Messspitze manuell zum Trokarpunkt zu verfahren und an dieser Stelle eine Registrierung auszulösen, wie es die US 6,201,984 B1 offenbart, in der ferner die weitere Bewegung und Positionierung eines Trokars an einem Ende eines Roboterhaltearmes beschrieben wird. Auch dieser Workflow ist jedoch aufwändig und fehleranfällig.

Ausgehend von diesem Stand der Technik ist es Aufgabe der vorliegenden Erfindung, eine schonende, an den gewohnten Arbeitsablauf vor und während eines chirurgischen Eingriffs angepasste Möglichkeit bereitzustellen, ein chirurgisches Instrument mit einem Haltearm zu verbinden und stabil im Körper eines Patienten zu halten.

Diese Aufgabe wird durch eine Andockvorrichtung mit den Merkmalen des Anspruchs 1 gelöst.

Die weitere Aufgabe, ein chirurgisches Instrument für einen chirurgischen Eingriff günstig vorzupositionieren und um einen korrekt registrierten Pivotpunkt bewegen zu können, wird durch die chirurgische Haltevorrichtung mit den Merkmalen des Anspruchs 10 gelöst. Noch eine weitere Aufgabe besteht darin, ein chirurgisches Instrument schnell und schonend in einer operationsgeeigneten Ausrichtung mit einem Haltearm einer chirurgischen Haltevorrichtung zu verbinden. Diese Aufgabe wird durch ein hier beschriebenes aber nicht beanspruchtes Verfahren gelöst. Dieses Verfahren ist nicht Teil der vorliegenden Erfindung.

Weiterbildungen sind in den jeweiligen Unteransprüchen ausgeführt.

Eine erste Ausführungsform einer Andockvorrichtung, die dazu ausgebildet ist, ein chirurgisches Instrument mit einem Haltearm einer chirurgischen Haltevorrichtung in einer Andocksituation zu verbinden, weist eine zweiteilige Adaptervorrichtung auf. Diese ist so gestaltet, dass sie an ihrem proximalen Ende mit dem Haltearm und an ihrem distalen Ende mit dem chirurgischen Instrument verbunden werden kann. Dabei hat das chirurgische Instrument einen stabförmigen Schaft und proximal daran anschließend eine Handhabe und ist dazu ausgebildet, durch einen Trokar geführt zu werden. Die zweiteilige Adaptervorrichtung weist einen ersten Adapter mit einem länglichen Abschnitt auf, dem entsprechend eine Längsachse zugehört; außerdem hat der erste Adapter einen Verbindungskörper. Der Verbindungskörper liegt an einem distalen Ende des ersten Adapters vor und weist ein erstes Eingriffselement mit einer endständigen Verbindungsfläche auf. Ferner weist die zweiteilige Adaptervorrichtung einen zweiten Adapter auf. Der zweite Adapter ist dazu ausgebildet, an dem Schaft des chirurgischen Instruments angeordnet zu werden. Er weist ein in der Andocksituation zu der endständigen Verbindungsfläche weisendes und mit dieser korrespondierendes Eingriffselement auf. Der Verbindungskörper des ersten Adapters bildet mit dem korrespondierenden Eingriffselement des zweiten Adapters in der Andocksituation eine lösbare Verbindung. Die endständige Verbindungsfläche des ersten Adapters ist in Bezug auf dessen Längsachse um einen vorbestimmten ersten Winkel, der in einem Bereich von 45° bis 75° liegt, geneigt.

In einer weiteren Ausführungsform der erfindungsgemäßen Ausführungsform kann der Neigungswinkel bevorzugt in einem Bereich von 55 ° bis 65 °, ganz besonders bevorzugt bei 60 ° liegen.

Die Begriffe "proximal" und "distal" beziehen sich auf die Lage gegenüber dem Bediener, sodass "proximal" also näher beim Bediener liegend und "distal" weiter entfernt vom Bediener liegend bedeutet.

Als "Andocksituation" wird dabei der Zustand bezeichnet, in dem über die erfindungsgemäße Andockvorrichtung der Haltearm vollständig mit dem chirurgischen Instrument verbunden ist und der damit zugleich die Ausgangssituation für eine Verwendung des chirurgischen Instruments in einem folgenden chirurgischen Eingriff darstellt.

Als chirurgische Haltevorrichtung werden hierin sowohl passive Haltevorrichtungen bezeichnet, die das chirurgische Instrument lediglich in einer voreingestellten Lage halten, als auch aktive Haltevorrichtungen, über die das chirurgische Instrument darüber hinaus bedienbar, insbesondere umpositionierbar ist, worunter auch komplette Chirurgie-Roboter zu verstehen sind, über die auch ferngesteuert ein chirurgischer Eingriff durchführbar ist. Das chirurgische Instrument kann im Speziellen ein Laparoskop sein, allgemein sind auch weitere chirurgische Instrumente darunter zu verstehen, die in der Mikrochirurgie und insbesondere in der Laparoskopie zum Einsatz kommen.

Vorteilhaft wird der zweite Adapter der zweiteiligen Adaptervorrichtung direkt an dem chirurgischen Instrument angebracht, was bereits in der Vorbereitung eines chirurgischen Eingriffs geschehen kann. Dadurch wird es im Anschluss deutlich leichter, das Instrument, das bereits mit dem zweiten Adapter versehen ist, an den ersten Adapter zu koppeln, da nur das erste Eingriffselement mit dem korrespondierenden Eingriffselement verbunden werden muss, was unter geringem Arbeitsaufwand und insbesondere unter geringstmöglicher Bewegung des Instruments selbst, folglich mit geringer Belastung des Patientengewebes erfolgt. Die Neigung der endständigen Verbindungsfläche hat diesen Vorteil insbesondere gegenüber dem Stand der Technik, der durch die DE 10 2014 116 103 A1 und EP 2 246 006 A2 gebildet wird, die beide horizontale Verbindungsfläche zwischen Haltebauteilen aufweisen und die Wahrung des Pivotpunktes an der Einstichstelle des Trokars dort nicht im Vordergrund steht. Mit der erfindungsgemäßen Lösung kann ein Endoskop unter Wahrung des festgelegten Pivotpunktes mit dem Haltearm direkt und zielgerichtet verbunden werden.

Der Verbindungskörper des ersten Adapters kann vollständig oder nur durch einen Teil des Verbindungskörpers das erste Eingriffselement bilden, oder das erste Eingriffselement ist daran angeordnet. So kann durch in Eingriff Bringen des ersten Eingriffselements des ersten Adapters mit dem korrespondierenden Eingriffselement des zweiten Adapters eine Verbindung hergestellt werden, die ohne Zuhilfenahme eines Werkzeugs lösbar ist.

Durch die um den vorbestimmten ersten Winkel - damit ist der kleinste zwischen Längsachse des länglichen Abschnitts des ersten Adapters und endständiger Verbindungsfläche des ersten Adapters eingeschlossene Winkel gemeint - geneigte endständige Verbindungsfläche wird letztlich die Ausrichtung des Schafts des chirurgischen Instruments festgelegt. "Endständig" bezieht sich auf das distale Ende des ersten Adapters und somit auf diejenige Seite des Verbindungskörpers, die von dem länglichen Abschnitt weg- weisend orientiert ist. Das korrespondierende Eingriffselement des zweiten Adapters wird beim Herstellen der Verbindung zwischen erstem und zweitem Adapter entsprechend der endständigen Verbindungsfläche ausgerichtet und folglich auch das chirurgische Instrument. Vorteilhaft kann so das chirurgische Instrument für einen chirurgischen Eingriff geschickt vorpositioniert werden. Hierbei meint "vorbestimmt", dass vorteilhaft je nach gewünschter, herbeizuführender Andocksituation, die bspw. von der Art des chirurgischen Eingriffs oder der Art des chirurgischen Instruments abhängen kann, Andockvorrichtungen mit größeren oder kleineren ersten Winkeln bereitgestellt werden können.

In einer weiteren Ausführungsform ist der Verbindungskörper mit dem Abschnitt, der die Verbindungsfläche aufweist, als Schlitten ausgebildet. Das korrespondierende Eingriffselement des zweiten Adapters ist korrespondierend als Schienenelement zur Aufnahme des Schlittens ausgebildet. Das bedeutet, dass die Verbindung von erstem und zweitem Adapter über eine Bewegung parallel zu der Verbindungsfläche erfolgt, parallel zu der Längsachse des Schienenelements. Der Schlitten kann dabei beispielsweise einen trapezförmigen oder schwalbenschwanzförmigen Querschnitt und das Schienenelement dazu korrespondierend eine Trapez- oder Schwalbenschwanzkontur aufweisen, sodass eine formschlüssige Verbindung entsteht. Natürlich können auch andere, dem Fachmannbekannte Geometrien bzw. Eingriffsquerschnitte als die Schwalbenschwanzform und das entsprechende Negativ dazu für Schlitten und Schiene gewählt werden. Schlitten und Schiene sind flach, bevorzugt mit einer Höhe von nur wenigen Millimetern bis zu einem Zentimeter ausgeführt.

Eine noch weitere Ausführungsform der erfindungsgemäßen Adaptervorrichtung sieht vor, dass der Verbindungskörper des ersten Adapters eine erste Öffnung aufweist. Die Öffnung kann, bzw. deren Ränder können in einem Winkel angefast, angeschnitten oder geneigt sein, der dem ersten Winkel entspricht, den die endständige Verbindungsfläche in Bezug auf die Längsachse des ersten Adapters aufweist. Anders ausgedrückt, verläuft eine imaginäre Achse durch die erste Öffnung parallel zu der Längsachse des Schienenelements und damit in der Andocksituation parallel zu der Längsachse des chirurgischen Instruments bzw. zu dessen Schaft.

Die erste Öffnung kann als Loch in der Wandung des Verbindungskörpers oder als Durchgangsöffnung oder als zylindrische Ausnehmung geformt sein, deren Ausrichtung parallel zu der endständigen Verbindungsfläche verläuft.

Die erste Öffnung dient dazu, einen Laserstrahl, worunter auch ein Bündel paralleler Laserstrahlen zu verstehen ist, aus einer Laservorrichtung in dem ersten Winkel austreten zu lassen, die an der Öffnung angeordnet ist. Wenn die Öffnung sogar einer zylindrischen Ausnehmung zugehört, kann diese die Laservorrichtung einhausen.

Außerdem weist der längliche Abschnitt des ersten Adapters eine zweite Öffnung auf, die in einem zweiten Winkel gegenüber der Längsachse des ersten Adapters geneigt ist. Die zweite Öffnung kann auch ein "Loch", ggfs. mit winklig angefasten Rändern, in der Wandung des ersten Adapters bzw, in dem Mantel eines zylindrischen Körpers des Adapters, oder eine zylindrische Ausnehmung hierin (ggfs. ebenfalls angefast) sein und ebenfalls eine Aufnahme für eine zweite Laservorrichtung bilden. Die zweite Öffnung dient ebenfalls dazu, einen Laserstrahl aus der zweiten Laservorrichtung in dem zweiten Winkel austreten zu lassen.

Der zweite Winkel ist dabei kleiner als der erste Winkel und liegt in einem Bereich von 20 ° bis 80 °. Entlang dieser Winkel gedachte Achsen sind so ausgerichtet, dass sie sich in einem vorbestimmten Schnittpunkt schneiden, der durch den ersten und zweiten Winkel, sowie den Abstand der beiden Öffnungen voneinander, gemessen entlang der Längsachse des länglichen Abschnitts des ersten Adapters, festgelegt wird, und insofern "vorbestimmt" ist, dass er abhängig von der Schaftlänge des zu verbindenden chirurgischen Instruments festgelegt wird.

Die Öffnungen, aus denen Laserlicht austreten kann, können auch für das verwendete Laserlicht transparente Fenster aufweisen. Die Öffnungen sind also dazu ausgebildet, dass Laservorrichtungen in den entsprechenden Winkeln von dem ersten Adapter abstrahlende Laserstrahlen aussenden können, die sich beide in einem vorbestimmten Schnittpunkt schneiden, der in einer Betriebsanordnung der Andockvorrichtung dem Pivotpunkt entspricht.

Durch den Punkt, in dem sich die Laserstrahlen aus den beiden Öffnungen schneiden, ist zugleich der Abstand des Schnittpunkts von der Andockvorrichtung und folglich von dem Haltearm festgelegt.

Jede der Laservorrichtungen kann eine Laserfaser sein, die mit einer Laserstrahlquelle verbunden werden kann. Die Laserstrahlquelle kann durchaus außerhalb der Andockvorrichtung liegen, so dass nur die Fasern innerhalb des Adapters geführt werden, die an der Öffnung Laserlicht austreten lassen.

Die Laservorrichtungen könnten dabei alternativ auch Lasermodule mit eigener Laserstrahlquelle sein, die den Laserstrahl selbst erzeugen.

Vorteilhaft sind die Fenster, die die Öffnungen verschließen, diffraktive Elemente, die den jeweiligen Laserstrahl bspw. kreuzförmig austreten lassen.

Als "Pivotpunkt" wird hierin der fixe Punkt im Raum bezeichnet, in dem der Trokar gehalten wird. Um diesen Pivotpunkt kann der Trokar bzw. kann das chirurgische Instrument geschwenkt werden. Translation entlang der Längsachse des Schafts und Rotation um diese sind davon unabhängig möglich. Bei einem chirurgischen Eingriff ist ein Soll-Pivotpunkt durch den Trokarpunkt, die Einstichstelle des Trokars in einen einzubringenden Körper, der ganz abstrakt ein beliebiger Volumenkörper sein kann, *in praxi* aber das Körpergewebe des Patienten sein wird, vorgegeben. Dieser Punkt wird jedoch verschoben, wenn das Bewegen des chirurgischen Instruments nicht korrekt auf diesen Soll-Pivotpunkt abgestimmt ist, sodass es zu Verletzungen an dem Gewebe rund um die Einstichstelle kommen kann. Die erfindungsgemäße Andockvorrichtung vermeidet dies vorteilhaft, indem der vorbestimmte Schnittpunkt so ausgerichtet werden kann, dass er im Pivotpunkt des Trokars liegt, durch den das gehaltene chirurgische Instrument geführt wird. Dadurch wird ein (von der Länge des Schafts des chirurgischen Instruments abhängiger) vorbestimmter Abstand des Haltearms zum Pivotpunkt eingestellt. Das chirurgische Instrument hat dabei eine feste Länge, wonach der Pivotpunkt sich nach dieser festen Länge bestimmt. Folglich kann nach dem vollständigen Verbinden das chirurgische Instrument um diesen Pivotpunkt bewegt werden.

Gemäß einer weiteren Ausführungsform grenzt die erste Öffnung an die endständige Verbindungsfläche an. Somit wird ein möglichst geringer Abstand zwischen der ersten Öffnung und dem mit dem zweiten Adapter verbundenen Schaft des chirurgischen Instrumentes erzielt, sodass der von der ersten Laservorrichtung ausgehende erste Laserstrahl nicht nur parallel, sondern nur mit einem geringen Versatz zu der Längsachse des Schafts des chirurgischen Instruments in der Andocksituation verläuft.

Dies wird auch durch den flach ausgeführten Schlitten und Schiene möglich, sodass die Instrumentenachse möglichst nah an der Laserachse der ersten Laservorrichtung liegt.

In einer weiteren Ausführungsform können in dem ersten Adapter zumindest eine elektrische Anschlussleitung und an der Oberfläche des proximalen Endes und/oder distalen Endes des ersten Adapters zumindest eine elektrische Kontaktfläche für die Laservorrichtung(en) bzw. den zweiten Adapter vorliegen.

Es ist denkbar, dass je Laservorrichtung eine elektrische Anschlussleitung und eine elektrische Kontaktfläche vorliegt.

Eine weitere Ausführungsform der erfindungsgemäßen Andockvorrichtung sieht vor, dass der zweite Adapter neben dem korrespondierenden Eingriffselement ein Rohrstück aufweist, wobei das korrespondierende Eingriffselement des zweiten Adapters in der Andocksituation über dem Rohrstück des zweiten Adapters angeordnet ist. Das Rohrstück weist eine zylindrische Durchtrittsöffnung auf, durch die der Schaft des chirurgischen Instruments führbar ist, wobei die Durchtrittsöffnung im Durchmesser nur unwesentlich größer als der Durchmesser des Schafts sein sollte, damit dieser in der Durchtrittsöffnung möglichst geringes Spiel hat. Die Längsachse der zylindrischen Durchtrittsöffnung des Rohrstücks verläuft dabei parallel zu dem korrespondierenden Eingriffselement, sodass nach Anordnen des zweiten Adapters an dem Schaft des chirurgischen Instruments ein Bewegen des zweiten Adapters entlang der Achse des Schienenelements einem Bewegen des Schafts entlang dessen Achse entspricht. Eine Bewegung zum Verbinden der beiden Adapter entlang der Längsachse des Schafts des chirurgischen Instruments hat sich als besonders vorteilhaft und schonend erwiesen, da diese den Freiheitsgrad nutzt, der ohnehin bei einem mittels Trokar geführten chirurgischen Instrument vorhandenen ist, und der Bediener dabei die Adapter und den Volumenkörper, in dem der Trokar vorliegt, bspw. einen Patienten, zugleich im Blick hat.

Generell ist das Handhaben der Adapter und damit der Andockvorrichtung in Bezug auf einen beliebigen Volumenkörper, der den Pivotpunkt aufweist, technisch-mechanisch zu verstehen, die Handhabung repräsentiert ein rein technisch-mechanisches Verfahren.

Ferner kann das Rohrstück des zweiten Adapters ein Kupplungselement aufweisen, das mit einem korrespondierenden Kupplungselement am proximalen Ende des Schafts des chirurgischen Instruments kuppelbar ist, sodass der zweite Adapter nicht von dem Schaft rutschen kann. Alternativ oder zusätzlich kann der zweite Adapter auch verschraubt bzw. aufgeschraubt, angeklemmt oder angeklebt werden.

In noch einer Ausführungsform weist der erste Adapter an seinem proximalen Ende ein zweites Eingriffselement auf, das dazu ausgebildet ist, mit einem korrespondierenden Eingriffselement des Haltearms lösbar verbunden zu werden. Damit eine solche direkte Verbindung des ersten Adapters möglich ist, muss die Anschlussstelle des vorgesehenen Haltearms in Bezug auf Verbindungsmechanismus und Abmessungen bekannt sein, damit das proximale Ende des ersten Adapters daran angepasst werden kann. Dies ist bei zugelassenen Assistenzsystemen üblicherweise der Fall.

Eine noch weitere Ausführungsform sieht vor, dass der erste Adapter vorteilhaft einen Haltekonus aufweist, der an dem proximalen Ende des ersten Adapters vorliegt und der sich zu einer proximalen Spitze hin verjüngt. Dieser Haltekonus kann nicht nur ein zweites Eingriffselement darstellen, das mit einem korrespondierenden Eingriffselement des Haltearms oder einem anderen korrespondierenden Verbindungsstück in Eingriff gebracht werden kann, er ist ferner so ausgeformt, dass mit seiner Spitze eine sterile Abdeckung (Drape), die den Haltearm umgibt, durchstoßen werden kann.

Das Durchstoßen eines vorliegenden sterilen Drapes ist nicht notwendig, wenn lediglich ein mechanisches Halten ohne Signalübertragung erfolgen soll, oder wenn das Drape einen speziellen Signalübertragungsadapter zur Anordnung an einem Roboterkopf aufweist, sodass die durch das Drape gebildete Barriere erhalten bleiben kann. Dann genügt es, wenn ein Spalt zwischen zweitem Eingriffselement des ersten Adapters und Haltearm verbleibt.

Die Andockvorrichtung weist in einer weiteren Ausführungsform ein Flanschstück auf, das dazu ausgebildet ist, an einem korrespondierenden Gegenflansch des Haltearms angeordnet zu werden. Der Gegenflansch kann ein Standard-Flansch des Haltearms sein, dessen Form dem Fachmann für die Gestaltung des Flanschstücks der Andockvorrichtung zur Anordnung daran bekannt ist; eine Schraubverbindung kann ausreichen. Durch das Flanschstück wird es möglich, den ersten Adapter an seinem proximalen Ende beliebig zu gestalten, ohne an die Form vorhandener Verbindungsmöglichkeiten des Haltearms gebunden zu sein; das Flanschstück wird dazu korrespondierend gestaltet. In einer bevorzugten Ausführungsform weist daher das Flanschstück eine konusförmige Ausnehmung auf, die zur Aufnahme des Haltekonus des ersten Adapters mit einer äußeren Umfangsform des Haltekonus korrespondiert. Auch zwischen Flanschstück und Haltekonus kann ein Spalt verbleiben, der gerade ausreichend breit ist, um ein steriles Drape einlagig darin aufzunehmen und einzuklemmen. In einer weiteren Ausführungsform kann der Haltekonus am Flanschstück vorliegen und das proximale Ende des ersten Adapters weist die Ausnehmung auf, in die der Haltekonus aufnehmbar ist.

Sämtliche mit der erfindungsgemäßen Andockvorrichtung herbeiführbaren Verbindungen, d. h. zwischen erstem Adapter und Haltearm bzw. am Haltearm angeordnetem Flanschstück, zwischen erstem Adapter und zweitem Adapter sowie zwischen zweitem Adapter und chirurgischem Instrument können manuell vorgenommen werden und sind ebenso manuell lösbar.

In weiteren Ausführungsformen sind diese Verbindungen durch Ausrichtungselemente so gestaltet, dass die miteinander verbindbaren, zueinander korrespondierenden Elemente nur in einer eindeutigen Ausrichtung in Eingriff gebracht werden können.

Die erfindungsgemäße Andockvorrichtung kann als Einmalprodukt ausgebildet sein. Ebenso kann sie vollständig oder teilweise, insbesondere die beiden Adapter der zweiteiligen Adaptervorrichtung, sterilisierbar, bevorzugt autoklavierbar sein.

Die Erfindung bezieht sich ferner auf eine chirurgische Haltevorrichtung mit zumindest einem Haltearm, der über die erfindungsgemäße Andockvorrichtung mit einem chirurgischen Instrument verbindbar ist bzw. in der Andocksituation mit diesem verbunden ist. Das chirurgische Instrument weist, wie zuvor beschrieben, einen stabförmigen Schaft sowie eine Handhabe auf und ist dazu ausgebildet, durch einen Trokar geführt zu werden. Vorteilhaft ist das chirurgische Instrument in der Andocksituation so mit der chirurgischen Haltevorrichtung verbunden, dass es für einen chirurgischen Eingriff geschickt vorpositioniert ist. Dabei kann es ausreichen, wenn die Haltevorrichtung das chirurgische Instrument nur in dieser voreingestellten Position hält. Es kann aber ebenso ein Bewegen des chirurgischen Instruments über die Haltevorrichtung erfolgen, sowohl manuelle als auch angetriebene Systeme sind umfasst. Die Haltevorrichtung kann mehrere Haltearme aufweisen, von denen nur ein Teil oder alle jeweils über eine erfindungsgemäße Andockvorrichtung mit einem chirurgischen Instrument verbunden sind.

In einer weiteren Ausführungsform ist die chirurgische Haltevorrichtung ein Chirurgie-Roboter mit einem Roboterkopf. Als Chirurgie-Roboter gilt hierbei eine Haltevorrichtung, die eine Steuerungsvorrichtung zum gesteuerten Bewegen des Haltearms aufweist; den Roboterkopf bildet das Ende des Haltearms, an den ein Endeffektor, auf den die Bewegungen der Kinematik übertragen werden sollen, anschließbar ist. Dabei kann die Steuerung des Haltearms ferngesteuert erfolgen, dann umfasst die Steuerungsvorrichtung zumindest ein Bedienelement, über das Bedienereingaben erfasst werden, oder sie läuft völlig automatisiert über eine entsprechende in der Steuerungsvorrichtung hinterlegte Programmierung ab.

Gemäß einer weiteren Ausführungsform der erfindungsgemäßen chirurgischen Haltevorrichtung ist die Steuerungsvorrichtung mit einer ersten Laservorrichtung und einer zweiten Laservorrichtung verbunden. Die Laservorrichtungen sind so zueinander ausgerichtet, dass sich die von diesen ausgestrahlten Laserstrahlen in einem vorbestimmten Schnittpunkt schneiden. In einer bevorzugten Ausführungsform sind daher die erste Laservorrichtung an der ersten Öffnung des ersten Adapters und die zweite Laservorrichtung an der zweiten Öffnung des ersten Adapters der Andockvorrichtung angeordnet, sodass sich die Strahlrichtung durch die Neigung der Öffnungen ergibt. Die beiden Laserstrahlen können zur besseren Unterscheidung zwei unterschiedliche Farben haben. Jede der Laservorrichtungen kann dabei eine Laserfaser, die mit einer Laserstrahlquelle verbunden werden kann, oder ein Lasermodul mit eigener Laserstrahlquelle sein.

Steuerbar meint hierbei, dass eine Wirkverbindung zwischen den Laservorrichtungen und der Steuerungsvorrichtung besteht, sodass beispielsweise die Laservorrichtungen durch die Steuerungsvorrichtung aktivierbar und deaktivierbar sind. Dazu kann in der Steuerungsvorrichtung ein Andockprogramm hinterlegt sein, über das Bewegungen des Haltearms während des Verbindungsvorgangs ausgeführt oder die manuelle Bewegung desselben erlaubt oder gesperrt werden können.

Der Chirurgie-Roboter kann auf einem mobilen Untersatz angeordnet und somit frei im Raum positioniert werden.

Ein Verfahren zum Verbinden eines Haltearms einer chirurgischer Haltevorrichtung in eine Andocksituation mittels einer erfindungsgemäßen Andockvorrichtung mit einem chirurgischen Instrument, das einen stabförmigen Schaft und eine Handhabe aufweist und dazu ausgebildet ist, über einen Trokar geführt zu werden, umfasst die folgenden Schritte:
a) Verbinden des ersten Adapters mit dem Haltearm,
b) Anordnen des zweiten Adapters an dem chirurgischen Instrument,
c) Führen des zweiten Adapters zu dem ersten Adapter durch Bewegen des chirurgischen Instruments, und Verbinden des zweiten Adapters mit dem ersten Adapter durch in Eingriff Bringen der endständigen Verbindungsfläche des ersten Eingriffselements des ersten Adapters mit dem ersten korrespondierenden Eingriffselement des zweiten Adapters, dadurch insgesamt Verbinden des Haltearms mit dem chirurgischen Instrument.

Dadurch kann auf einfachste Weise die Verbindung hergestellt werden, durch die das Halten des chirurgischen Instruments ermöglicht wird, das direkt mit einer bestimmten Eindringtiefe (abhängig von der Länge des Schafts) und um den vorbestimmten ersten Winkel geneigt positioniert ist. Dabei spielt es an sich keine Rolle, in welcher Reihenfolge Schritte a) und b) ausgeführt werden, solange dies vor Schritt c) erfolgt.

In einer Weiterbildung des Verfahrens werden nach Schritt b), aber vor Schritt c), die folgenden Schritte ausgeführt:
b1) Positionieren des chirurgischen Instruments durch Einführen des Schafts in den Trokar, der um einem vorbestimmten/fixen Pivotpunkt schwenkbar gelagert ist,
b2) mittels der an der ersten Öffnung angeordneten Laservorrichtung Aussenden eines ersten Laserstrahls in einer vorbestimmten Richtung und Ausrichten des ersten Laserstrahls auf den Pivotpunkt durch Verschieben des Haltearms und damit des ersten Adapters,
b3) mittels der an der zweiten Öffnung des ersten Adapters angeordneten zweiten Laservorrichtung Aussenden eines zweiten Laserstrahls in eine vorbestimmte Richtung, der den ersten Laserstrahl in einem vorbestimmten Schnittpunkt schneidet, und Ausrichten des zweiten Laserstrahls auf den Pivotpunkt durch Verschieben des Haltearms parallel zu dem ersten Laserstrahl, dadurch insgesamt Ausrichten des ersten Adapters und folglich des Haltearms auf den Pivotpunkt,

Schritt a) kann optional auch erst nach Schritt b1) durchgeführt werden, wobei die übrigen Schritte bevorzugt in der dargestellten Reihenfolge auszuführen sind.

Diese entspricht dem üblichen Arbeitsablauf bei einem durch Haltevorrichtungen unterstützten mikrochirurgischen Eingriff, bei dem das Instrument erst in einen Trokar eingeführt und so dessen Pivotpunkt festgelegt wird, ehe es an einem Haltearm befestigt wird. Der Pivotpunkt wird durch die Lagerung des Trokars vorgegeben, wobei um den Pivotpunkt ein Schwenken bzw. Kippen des chirurgischen Instruments, bei dem der Trokar mit geschwenkt bzw. gekippt wird, möglich ist.

Schritte a) bis b1) werden manuell ausgeführt, wohingegen das Verschieben des Haltearms in Schritten b2) und b3) manuell oder durch elektronische Steuerung erfolgen kann. Beim Ausrichten des zweiten Laserstrahls auf den Pivotpunkt in Schritt b3) wird die Ausrichtung des ersten Laserstrahls auf den Pivotpunkt beibehalten, indem der Haltearm, der bereits mit dem ersten Adapter verbunden ist, lediglich noch parallel zu dem ersten Laserstrahl, also in der Richtung, in die der Laserstrahl verläuft, bewegt wird. Danach wird die Position des Haltearms mit erstem Adapter nicht mehr verändert. Somit liegt in Schritt c) der Schnittpunkt der Laserstrahlen im Pivotpunkt und damit zugleich in einem vorgegebenen Abstand zu dem Haltearm und zu dem ersten Adapter. Das chirurgische Instrument, das nun angeschlossen wird und vorpositioniert ist, kann davon ausgehend über von dem Haltearm übertragene Bewegungen umpositioniert werden.

Nach Verbinden des Haltearms mit dem chirurgischen Instrument können die Laserstrahlen wieder abgeschaltet werden.

Das Verfahren kann ferner vor Schritt a) Schritt a0) umfassen, nämlich das Anbringen eines Flanschstücks der Andockvorrichtung an dem Haltearm bzw. einem daran angebrachten Gegenflansch, sodass der erste Adapter nicht direkt mit dem Haltearm verbunden werden muss, sondern das Verbinden durch Eingreifen in das Flanschstück erfolgt.

In einer weiteren Ausführungsform kann beim Verbinden des ersten Adapters in Schritt a) mit der Spitze des ersten Adapters ein zumindest den Haltearm umhüllendes steriles Drape durchstoßen werden, sodass eine Kontaktierung, auch elektrisch, unter Aufrechterhaltung eines sterilen Operationsraums möglich ist. Ferner kann ein solches Drape auch zwischen Haltearm bzw. daran angeordnetem Flanschstück und erstem Adapter eingeklemmt werden.

In einer weiteren Ausführungsform des Verfahrens wird das chirurgische Instrument mit einem Haltearm eines erfindungsgemäßen Chirurgie-Roboters mit Steuerungsvorrichtung verbunden.

Bevorzugt werden dann zusätzlich folgende Schritte ausgeführt:
b2'), nach Schritt b2) in der Steuerungsvorrichtung Sperren von Bewegungen des Haltearms, die nicht parallel zu dem ersten Laserstrahl erfolgen,
b3'), nach Schritt e) Registrieren des Pivotpunktes durch Registrieren des Schnittpunkts des ersten und zweiten Laserstrahls in der Steuerungsvorrichtung, und Hinterlegen des registrierten Pivotpunkts in der Steuerungsvorrichtung, um Bewegungen des Haltearms in Bezug auf den registrierten Pivotpunkt angesteuert auszuführen.

Vorteilhaft wird durch Schritt b3') der Pivotpunkt, auf den der Haltearm ausgerichtet wird, in der Steuerungsvorrichtung hinterlegt, sodass der Chirurgie-Roboter einen Bezugspunkt gegenüber einem externen Körper erhält und folglich Bewegungen um diesen herum koordinieren kann. Der externe Körper kann ein Patientenkörper für einen chirurgischen Eingriff sein. Durch die präzise Vorpositionierung stimmt der registrierte Pivotpunkt mit dem Soll-Pivotpunkt überein, Schäden am Patientengewebe werden somit reduziert. Ein geringer systemimmanenter Fehler aufgrund dessen, dass der erste Laserstrahl nicht exakt den Verlauf des Instrumentenschafts abbildet, sondern in einem - wenngleich geringen - Versatz dazu verläuft, kann kalkulatorisch ausgeglichen werden.

Durch den optionalen Schritt b2') wird das Bewegen des Haltearms parallel zu dem ersten Laserstrahl vereinfacht und sichergestellt, dass die Ausrichtung des ersten Laserstrahls auf den Pivotpunkt in den weiteren Schritten beibehalten wird.

Diese und weitere Ausführungsformen der Andockvorrichtung und des Verfahrens sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine perspektivische Ansicht der Andockvorrichtung, verbunden mit einem Gegenflansch eines Haltearms und mit einem chirurgischen Instrument,
- **Fig. 2**: eine perspektivische Ansicht der Andockvorrichtung, verbunden mit einem Gegenflansch eines Haltearms,
- **Fig. 3**: eine perspektivische Ansicht des ersten Adapters und des Flanschstücks der Andockvorrichtung, verbunden mit einem Gegenflansch eines Haltearms,
- **Fig. 4**: eine weitere perspektivische Ansicht des ersten Adapters und des Flanschstücks der Andockvorrichtung, verbunden mit einem Gegenflansch eines Haltearms,
- **Fig. 5**: eine perspektivische Ansicht des ersten Adapters,
- **Fig. 6**: eine Längsschnittansicht durch den ersten Adapter und das Flanschstück der Andockvorrichtung, verbunden mit dem Gegenflansch des Haltearms,
- **Fig. 7**: eine Detailansicht des zweiten Adapters,
- **Fig. 8**: eine weitere Detailansicht des zweiten Adapters,
- **Fig. 9**: den zweiten Adapter, angeordnet an einem chirurgischen Instrument,
- **Fig. 10**: eine Detail-Teilansicht des proximalen Endes eines Schafts eines chirurgischen Instruments, an dem der zweite Adapter der Andockvorrichtung angebracht werden kann,
- **Fig. 11**: in a) bis g) mehrere Zwischenschritte beim Verbinden eines chirurgischen Instruments mittels der Andockvorrichtung mit einem Haltearm.

Die Erfindung umfasst eine Andockvorrichtung mit einer zweiteiligen Adaptervorrichtung, wobei der erste Adapter direkt oder über ein Flanschstück der Andockvorrichtung mit einem Haltearm einer chirurgischen Haltevorrichtung verbunden werden kann, und der zweite Adapter mit einem chirurgischen Instrument, sodass dieses von dem Haltearm gehalten wird, wenn der erste und der zweite Adapter miteinander in Eingriff stehen. Die Erfindung bezieht sich außerdem auf eine chirurgische Haltevorrichtung, die über die Andockvorrichtung mit einem chirurgischen Instrument verbunden ist, sowie auf ein Verfahren zum Verbinden des chirurgischen Instruments mit dem Haltearm mittels der erfindungsgemäßen Andockvorrichtung. Der Haltearm kann einem Chirurgie-Roboter angehören.

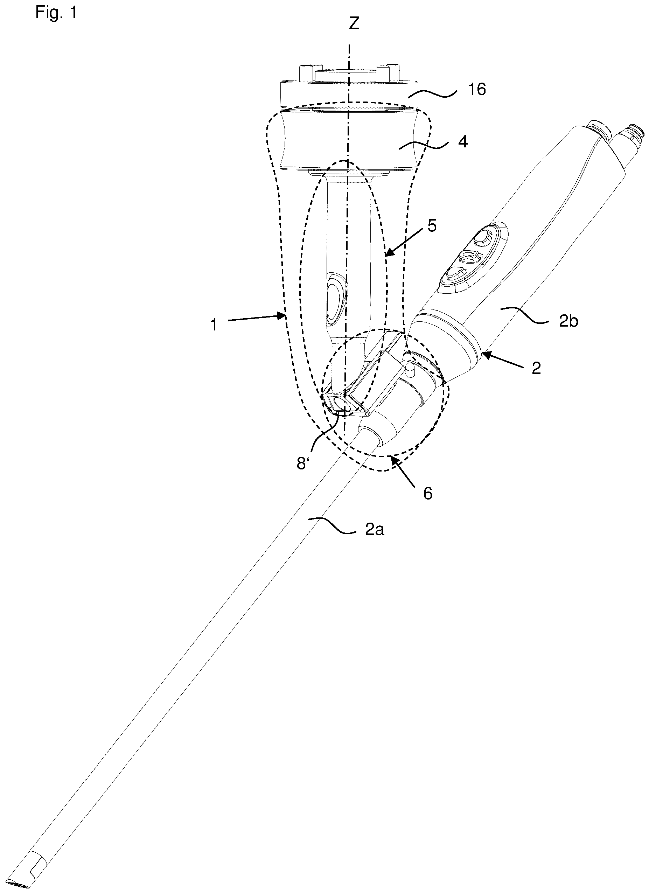

**Fig. 1** bis **Fig. 10** zeigen eine Ausführungsform der erfindungsgemäßen Andockvorrichtung 1 in verschiedenen verbundenen und unverbundenen Zuständen der einzelnen Teile. Der Haltearm der Haltevorrichtung, an den die Andockvorrichtung 1 das chirurgische Instrument 2 andockt, ist dabei nicht figurativ dargestellt. Stattdessen sind jeweils noch die Verschraubungen 17 zu sehen, über die ein Gegenflansch 16 an dem Haltearm befestigt wird, die in der Praxis natürlich in entsprechenden Bohrungen oder sonstigen Anschlussstellen des Haltearms bzw. Roboterkopfs, wenn es sich um den Haltearm eines Chirurgie-Roboters handelt, verschwinden.

**Fig. 1** zeigt (ohne Darstellung des Haltearms) die Andockvorrichtung 1 im vollständig verbundenen Zustand, an einem proximalen Ende mit dem Gegenflansch 16 eines Haltearms über ein Flanschstück 4 verbunden, an einem distalen Ende mit einem chirurgischen Instrument 2 verbunden. Der erste Adapter 5 ist mit dem Flanschstück 4 und mit dem zweiten Adapter 6 verbunden. In diesem Zustand ist das chirurgische Instrument 2 also bereit, durch den Haltearm gehalten und ggf. auch umpositioniert zu werden.

Das chirurgische Instrument 2, das mit der Andockvorrichtung 1 an den Haltearm gekoppelt werden soll, weist einen länglichen Schaft 2a auf, der an seinem proximalen Ende mit einer Handhabe 2b verbunden ist und an dessen distalem Ende ein Werkzeug (nicht dargestellt) wie einen Manipulator oder auch eine Kamera (Endoskop) vorliegen kann. Über den Schaft 2a verlaufen die entsprechenden Verbindungen zur mechanischen und/oder elektrischen Kontaktierung und Handhabung des Werkzeugs zur Handhabe 2b.

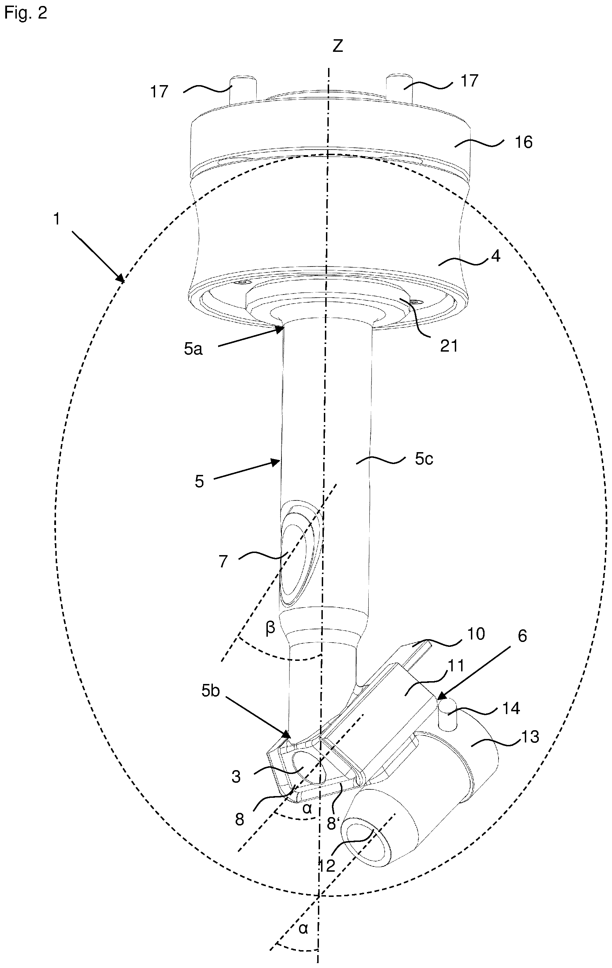

In **Fig. 2** ist die Andockvorrichtung 1 zur Verdeutlichung ohne chirurgisches Instrument 2 dargestellt, wobei dennoch der erste Adapter 5 mit dem zweiten Adapter 6 verbunden ist, ein Zustand, der üblicherweise nicht hergestellt wird, da stets der zweite Adapter 6 mit dem chirurgischen Instrument 2 verbunden wird, ehe er an den ersten Adapter 5 andockt.

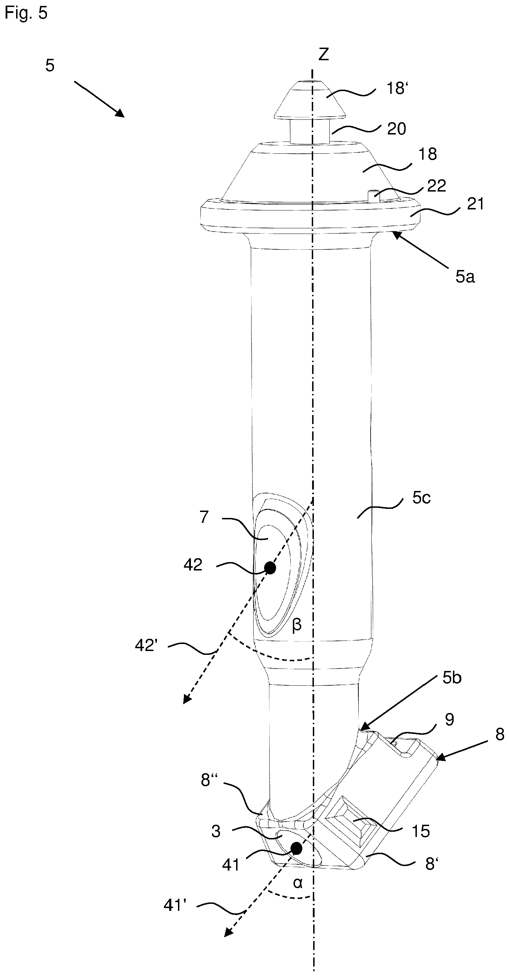

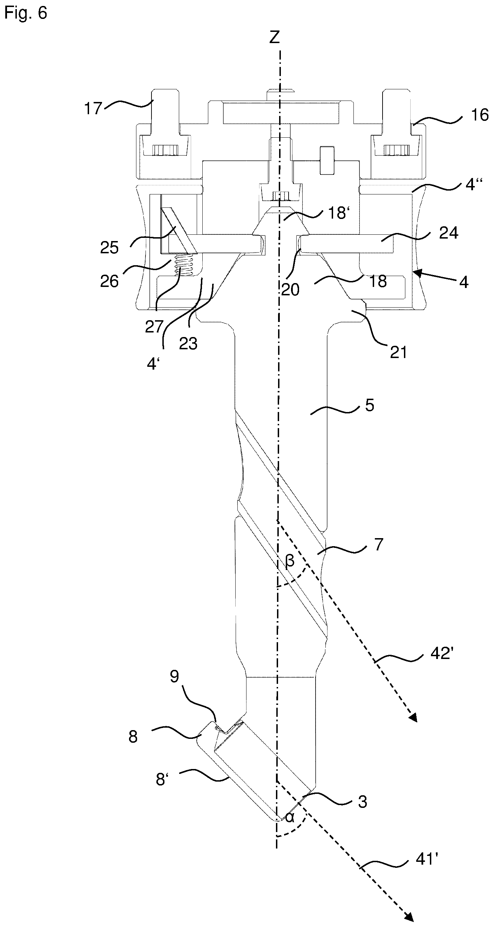

In der **Fig. 2** ist die Andockvorrichtung 1 mit einem speziell für die Aufnahme des Haltekonus 18, der in **Fig. 5** und **6** gezeigt ist, des ersten Adapters 5 ausgebildeten Flanschstück 4 verbunden dargestellt. Dieses Flanschstück 4 wird auf einfache Weise beispielsweise durch Verschraubungen 17 an den korrespondierenden (Standard-)Anschlüssen eines Gegenflansches 16 des Haltearms angebracht, sodass das Flanschstück 4 das korrespondierende Eingriffselement, in den gezeigten Ausführungsformen die Ausnehmung 23, für das proximale Ende 5a des ersten Adapters 5 bereitstellt.

Der Haltearm kann aber ebenso einen standardisierten Flansch aufweisen, solche sind insbesondere für verschiedene Chirurgie-Roboter-Systeme bekannt und liegen am Roboterkopf des jeweiligen Haltearms vor, an dem ein für ein passend ausgebildetes erstes Eingriffselement des ersten Adapters korrespondierendes Eingriffselement des Haltearms vorliegt, sodass der erste Adapter direkt angekoppelt werden kann.

In **Fig. 2****,** **3****,** **4** **und** **5** gezeigt, hat der erste Adapter 5 ein proximales Ende 5a zur Verbindung mit dem Haltearm und ein distales Ende 5b zur Verbindung mit dem zweiten Adapter 6, und dazwischen einen länglichen Abschnitt 5c. An dem distalen Ende 5b liegt dazu ein Verbindungskörper 8 vor, der sich von einer zum länglichen Abschnitt 5c weisenden Seite 8" hin zu einer endständigen Verbindungsfläche 8' verbreitert und somit eine Art Schlitten oder Schwalbenschwanz mit trapez- bzw. schwalbenschwanzförmigem Querschnitt bildet. Somit wird ein erstes Eingriffselement des ersten Adapters 5 durch den Verbindungskörper 8 bereitgestellt. Alternative, nicht dargestellte Ausführungsformen können das erste Eingriffselement als Teilstück des Verbindungskörpers oder daran angeordnetes Element vorsehen. Der Verbindungskörper 8 passt mit seiner Verbindungsfläche 8' in ein korrespondierendes Eingriffselement 11 am zweiten Adapter 6, das hier eine Schlittenaufnahme in Form eines Schienenelements 11 ist.

Es ist denkbar, dass die zueinander korrespondierenden Elemente vertauscht vorliegen, also der erste Adapter 5 ein Schienenelement und der zweite Adapter 6 einen Schlitten aufweist. Es versteht sich, dass die dargestellten Verbindungsmechanismen lediglich beispielhaft für andere, jeweils zueinander korrespondierende Eingriffselemente sind.

Der erste Adapter 5 umfasst ferner eine Längsachse Z des länglichen Abschnitts 5c, die nach Ankopplung an den Haltearm (in einem 90 °-Winkel gegenüber dessen Stirnseite) wie dargestellt vertikal verlaufen kann, das entspricht einer häufig anzutreffenden Kopplungssituation, aber auch von der Vertikalen abweichen kann. Beim Verbinden des ersten Adapters 5 mit dem Haltearm wird somit eine eindeutige Orientierung dieser Längsachse Z festgelegt.

Der gesamte Verbindungskörper 8 und somit insbesondere seine endständige Verbindungsfläche 8' ist um den Winkel α gegenüber der Längsachse Z des länglichen Abschnitts 5c abgewinkelt angeordnet, sodass die Verbindung mit dem zweiten Adapter 6 in eben diesem Winkel erfolgt. Dadurch wird ein fester Verbindungswinkel letztlich des chirurgischen Instruments 2 mit dem Haltearm hergestellt. Andockvorrichtungen 1 können mit unterschiedlichen Winkeln bereitgestellt werden; für die dargestellte Andocksituation hat sich ein Winkel in einem Bereich von 30 ° bis 90 °, erfindungsgemäß in einem Bereich von 45 ° bis 75 °, insbesondere von etwa 60 ° oder 45 ° als sinnvoll erwiesen, wodurch das chirurgische Instrument 2 in eine übliche Lage für den Einsatz in einem laparoskopischen Eingriff gebracht wird.

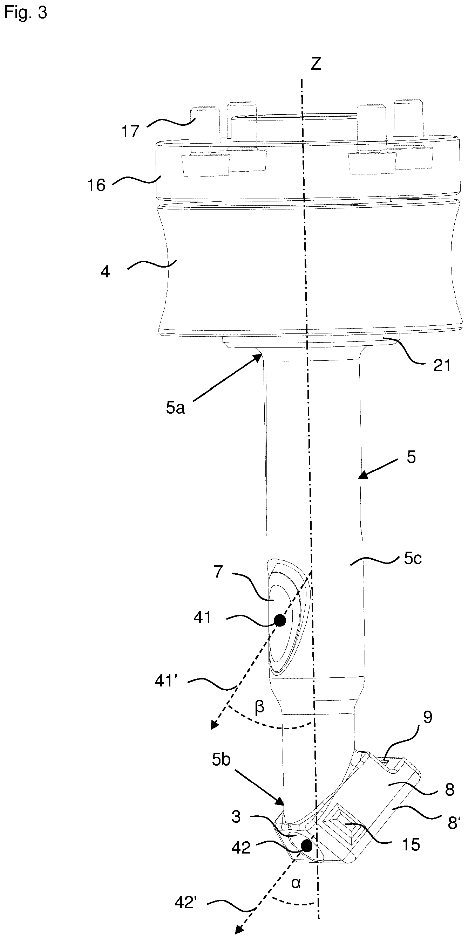

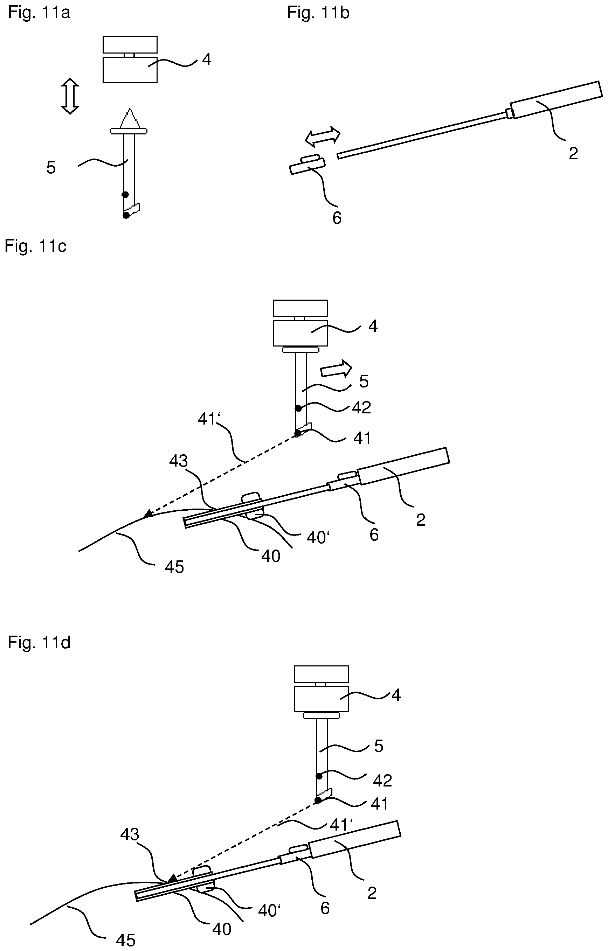

In den **Fig. 2****,** **3** **und** **5** zeigt der Verbindungskörper 8 des ersten Adapters 5 eine erste Öffnung 3 für den Austritt eines ersten Laserstrahls 41'. In den Verbindungskörper 8 an der ersten Öffnung 3 ist hier eine erste Laservorrichtung 41 bspw. in Form einer Laserfaser (in **Fig. 3** **und** **5** als Punkt gezeigt) integriert. Die erste Öffnung 3 kann über ein transparentes Fenster, hier figurativ nicht gezeigt, verschlossen sein. Die erste Öffnung 3 ist in einem ersten Winkel α geneigt, wobei in den **Fig. 2****,** **3** **und** **5** dazu die erste Öffnung 3 als Durchgangsöffnung mit angefasten Rändern gezeigt ist. Zum besseren Verständnis ist in **Fig. 3** **und** **5** der Laserstrahl 41' nach hinten gestrichelt bis zu Längsachse Z durchgezogen, um den Winkel α aufzuzeigen. Der Laserstrahl 41' strahlt entlang dem Neigungswinkel der ersten Öffnung 3 in einer um den ersten Winkel α gegenüber der Längsachse Z abgewinkelten Richtung von dem ersten Adapter 5 weg, also parallel zu der endständigen Verbindungsfläche 8' und parallel zu der Verbindungsrichtung zwischen Verbindungskörper 8 des ersten Adapters 5 und Schienenelement 11 des zweiten Adapters 6. In seinem länglichen Abschnitt 5c hat der erste Adapter 5 eine zweite Öffnung 7 für eine zweite Laservorrichtung 42 wie soeben für die erste Laservorrichtung 41 beschrieben, die einen zweiten Laserstrahl 42' aussendet. Die zweite Öffnung 7 ist wie auch die erste Öffnung 3 in den Figuren als Durchgangsöffnung gezeigt. Um den Winkel β in **Fig. 3** **und** **5** darzustellen, ist der zweite Laserstrahl 42' ebenfalls bis zur Längsachse Z des Adapters 5 gestrichelt verlängert. Der zweite Laserstrahl 42' strahlt in Neigungsrichtung der zweiten Öffnung 7 in einer um den zweiten Winkel β gegenüber der Längsachse Z abgewinkelten Richtung vom ersten Adapter 5 weg, wobei der zweite Winkel β kleiner ist als der erste Winkel α. Der zweite Laserstrahl 42' verläuft dabei in der Ebene, die durch die Längsachse Z und den ersten Laserstrahl 41' festgelegt ist und schneidet den ersten Laserstrahl 41' in einem vorbestimmten Schnittpunkt 44. Deren Funktion wird im Zusammenhang mit den Figuren **11a** bis **11g** näher beschrieben.

Aus der Schnittansicht in **Fig. 6****,** in der die Öffnungen 3, 7 Durchgangsöffnungen sind, wird deutlich, dass sich in diesem Fall die Richtung der Laserstrahlen 41', 42' durch die Ausrichtung der Öffnungen 3, 7 ergibt.

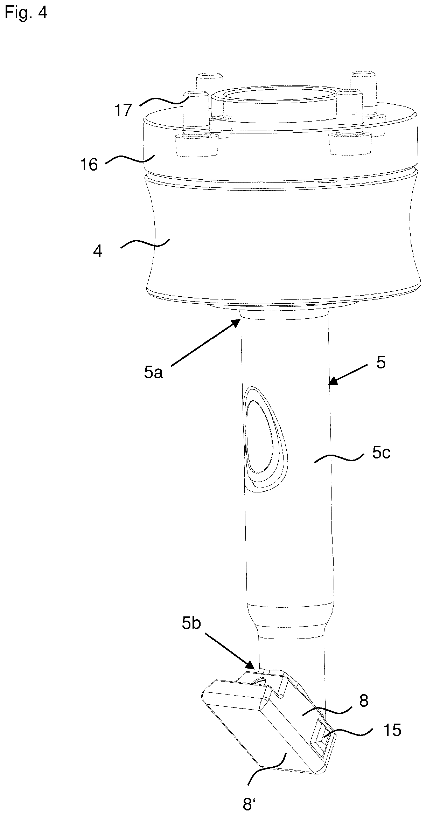

**Fig. 3** **und** **4** zeigen den ersten Adapter 5, wie er für die lösbare Verbindung mit dem zweiten Adapter 6 vorliegen sollte, nämlich bereits mit dem Flanschstück 4 verbunden und bereit, über den Verbindungskörper 8, an dem das erste Eingriffselement vorliegt oder der selbst das erste Eingriffselement bildet, mit dem Schienenelement 11 des zweiten Adapters 6 in Eingriff gebracht zu werden.

In **Fig. 5** und **6** ist am proximalen Ende 5a des ersten Adapters 5 als zweites Eingriffselement der Haltekonus 18 ausgeformt, der sich vom länglichen Abschnitt 5c aus in eine Spitze 18' verjüngt. An seiner breiten Seite wird der Haltekonus 18 durch einen umfänglich überstehenden Kragen 21 begrenzt. Der Haltekonus 18 dient zur Verbindung mit einem an dem Gegenflansch 16 des Haltearms angebrachten Flanschstück 4, dessen konusförmige Ausnehmung 23 den Haltekonus 18 aufnehmen kann. Der Haltekonus 18 weist eine Ausnehmung in Form einer umlaufenden Ringnut 20 auf.

Die Verbindung mit einem speziell für die Adaptervorrichtung 1 ausgebildeten Flanschstück 4 ist in der Schnittansicht in **Fig. 6** gezeigt. Das Flanschstück 4 weist einen mit dem Gegenflansch 16 verbundenen, bspw. angeschraubten Flanschkörper 4' auf. In dem Flanschkörper 4' liegt die konusförmige Ausnehmung 23 vor, die mit der Form des Haltekonus 18 korrespondiert. Eine oder mehrere beweglich gelagerte Halteschiene(n) 24 ragen in die Ausnehmung 23 hinein und werden über eine Führung 25 geführt. Wird der Haltekonus 18 des ersten Adapters 5 in die Ausnehmung 23 eingeführt, wird dabei die Halteschiene 24 von der Spitze 18' nach außen und eine gegenüber dem Flanschkörper 4' bewegliche Hülse 4" nach unten geschoben. Wenn der Haltekonus 18 so weit eingeschoben ist, dass die Ringnut 20 auf der Höhe der Halteschiene 24 liegt, kann die Halteschiene 24 sich wieder nach innen bewegen und verriegelt damit den Haltekonus 18 und folglich den ersten Adapter 5 an dem Flanschstück 4. Eine mit einem Stift 27 geführte Feder 26 bewirkt die Rückstellung der Hülse 4" und der Halteschiene 24. Zum Lösen des ersten Adapters 5 kann umgekehrt vorgegangen werden, also durch Angreifen an der Hülse 4" die Halteschiene 24 aus der Ringnut 20 gezwungen und somit der Weg des Haltekonus 18 aus der Ausnehmung 23 heraus freigegeben werden.

Ferner ist an dem überstehenden Kragen 21 ein Ausrichtungsstift 22 angeordnet, der in Richtung der Spitze 18' weist. Dieser soll in eine (auch in der Schnittansicht nicht sichtbare) stiftförmige Ausnehmung an dem Flanschstück 4 eingeführt werden, sodass der erste Adapter 5 nur in einer eindeutigen Ausrichtung gegenüber dem Flanschstück 4 und damit dem Haltearm verbunden werden kann. Diese oder vergleichbare Ausrichtungshilfen für eine eindeutige Ausrichtung sind optional und vereinfachen den Verbindungsvorgang weiter.

Der Haltearm, und das Flanschstück 4, falls dieses als Teil der Andockvorrichtung 1 verwendet werden soll, können für ein steriles Operationssetting von einem sterilen Drape (Einweghülle) umhüllt sein. Damit dennoch das Ankoppeln des ersten Adapters 5 problemlos möglich ist, kann die Spitze 18' des Haltekonus 18 beim Einführen in die konusförmige Ausnehmung 23 das Drape durchstoßen, sodass der Weg zum Flansch freigeben wird. Die Spitze 18' ist dennoch leicht abgerundet, um Verletzungen des medizinischen Personals bei der Handhabung zu vermeiden. Somit ist nicht nur eine rein mechanische Verbindung möglich, sondern es können auch elektrische Kontaktstellen im Haltekonus 18 vorliegen, die dann entsprechend von Teilen des Haltearms kontaktiert werden und für eine elektrische bzw. elektronische Ankopplung sorgen. Diese können bspw. Energieversorgungsleitungen für die Laservorrichtungen 41, 42 sein. Der Abschnitt des Drapes um den Haltekonus 18 des ersten Adapters 5 kann auch in einem schmalen Spalt, der zwischen erstem Adapter 5 und Flanschstück 4 verbleibt, aufgenommen werden. Die übrigen Teile der Andockvorrichtung 1, insbesondere der erste und der zweite Adapter 5, 6, befinden sich im sterilen Raum und müssen daher selbst steril sein. Die Andockvorrichtung 1 ist daher als Einmal-Produkt, das nach dem Einsatz entsorgt wird, oder als sterilisierbares Mehrweg-Produkt gestaltet, wobei zumindest die zweiteilige Adaptervorrichtung sterilisierbar ist.

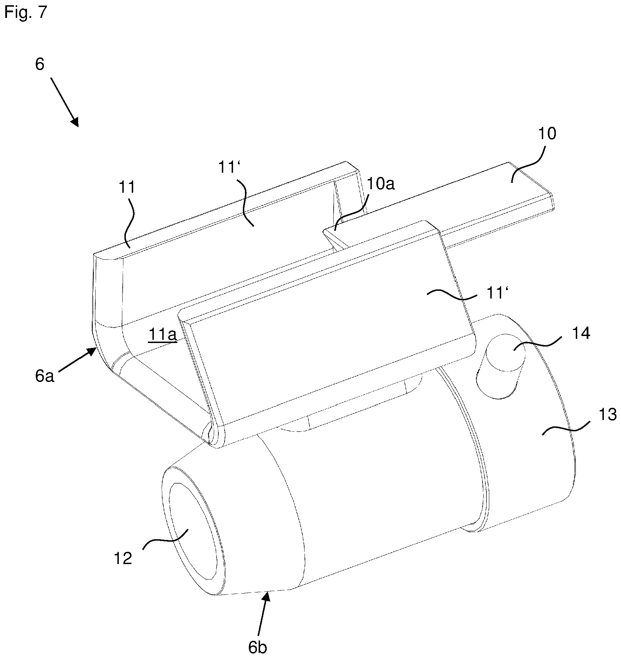

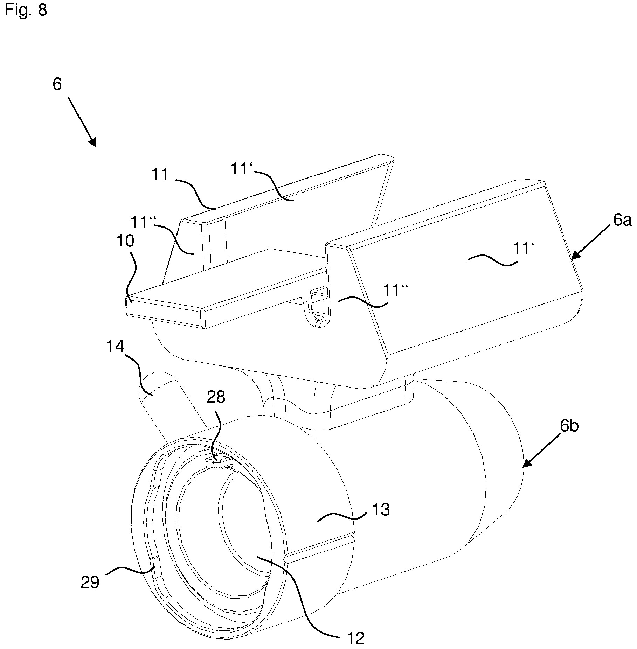

**Fig. 7** und **Fig. 8** zeigen, dass der zweite Adapter 6 einen Schienenabschnitt 6a und ein Rohrstück 6b aufweist. Am Schienenabschnitt 6a liegt ein Schienenelement 11 vor, das in der Andocksituation nach oben, d. h. zur endständigen Verbindungsfläche 8' des Verbindungskörpers 8 des ersten Adapters 5 weist und zu dieser Seite hin geöffnet ist. Zu der geschlossenen, unteren Seite schließt das Rohrstück 6b an. Das Schienenelement 11 korrespondiert derart mit der endständigen Verbindungsfläche 8', dass es einen Aufnahmeraum 11a für den Verbindungskörper 8 des ersten Adapters 5 bildet. Dazu laufen die Seitenwände 11' des Schienenelements 11 zu dem Rohrstück 6b des zweiten Adapters 6 hin auseinander, sodass eine trapez- bzw. schwalbenschwanzförmige Kontur des Schienenelements 11 vorliegt. Somit können die Adapter 5,6 nur entlang einer Längsachse des Schienenelements 11 miteinander verbunden werden. Dazu wird der Verbindungskörper 8 von einem Längsende des Schienenelements 11 in den Aufnahmeraum 11a geschoben, sodass der erste Adapter 5 und der zweite Adapter 6 eindeutig zueinander ausgerichtet sind. Eine um 180° verdrehte Verbindung ist nicht möglich, weil nur ein Längsende des Schienenelements 11 freiliegt, um den ersten Adapter einzuschieben, wenn der zweite Adapter 6 bereits an das chirurgische Instrument 2 angeordnet ist. Darüber hinaus können die Seitenwände 11' zu diesem freiliegenden Längsende hin auseinanderlaufen, um das Einschieben zu erleichtern. An einem zweiten Längsende des Schienenelements 11 wird dieses durch Rückwände 11" begrenzt, sodass der Schlitten 8 nicht hindurchrutscht. Lösen der Verbindung erfolgt schlicht durch Längsverschieben in die entgegengesetzte Richtung.

An dem zweiten Längsende des Schienenelements 11 ist ein Sicherungsclip 10 mit einer zum Aufnahmeraum 11a weisenden Nase 10a angeordnet, die in eine entsprechende Einkerbung 9 am Verbindungskörper 8 des ersten Adapters 5 zur Sicherung gegen ungewolltes Lösen eingreifen kann. Um die Sicherung zu lösen, kann Druck auf den Sicherungsclip 10 ausgeübt werden, der sich dann aufbiegt, sodass die Nase 10a aus der Einkerbung 9 gehoben wird. Am Verbindungskörper 8 des ersten Adapters 5 können Sicherungsauskragungen 15 vorliegen, die zusätzlich für einen festen Halt innerhalb des Schienenelements 11 sorgen können.

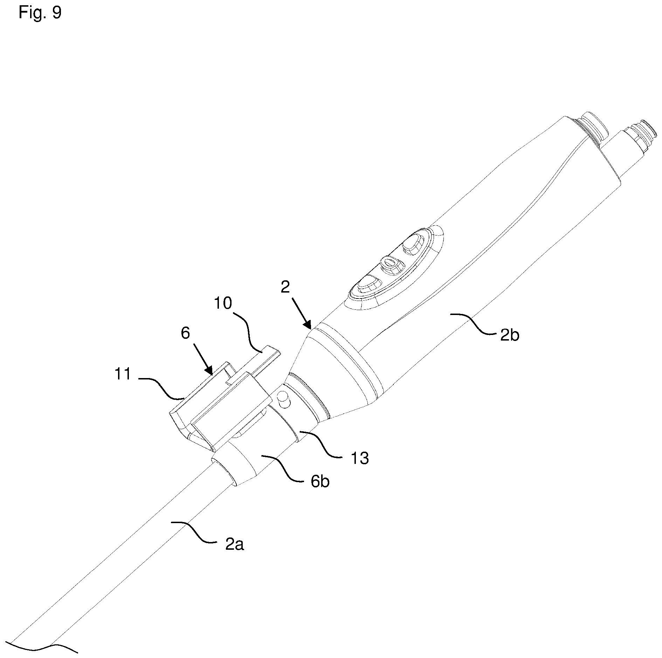

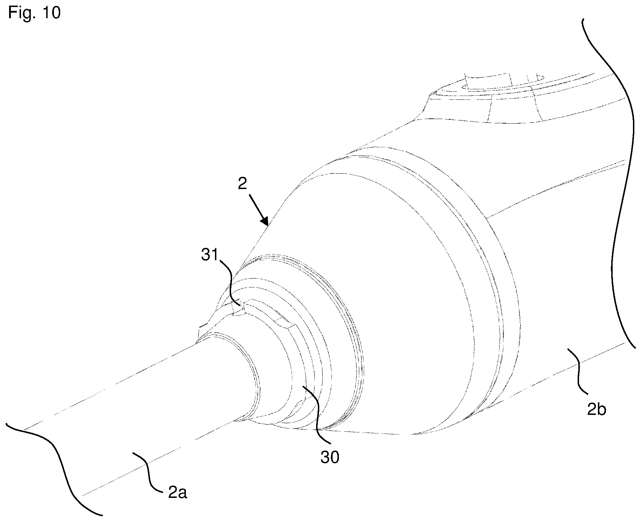

An den Schienenabschnitt 6a schließt sich ein Rohrstück 6b an, das eine Durchgangsöffnung 12 aufweist, die parallel zu dem Schienenelement 11 verläuft, genauer parallel zu dessen Längsachse, entlang der die beiden Adapter 5,6 miteinander verbunden werden. In **Fig. 8** ist gezeigt, dass am distalen Ende des Rohrstücks 6b ein Kupplungselement 13 in Form eines Verbindungsrings mit einem Greifnippel 14 vorgesehen ist, wobei der Verbindungsring 13 umfänglich innenseitig eine geformte Ausnehmung 29 aufweist, sowie einen Ausrichtungsstift 28. Der Verbindungsring 13 ist drehbar, wobei der Greifnippel 14 die Drehung des Verbindungsrings 13 erleichtert, um den Adapter an das Endoskop anzukoppeln. Der Greifnippel 14 kann auch einen Stift aufweisen, um im Inneren des Verbindungsrings 13 die Führung der Kupplung zu unterstützen und den Rotationsradius des Verbindungsrings 13 zu begrenzen. Um den zweiten Adapter 6 mit dem chirurgischen Instrument 2 zu der in **Fig. 9** gezeigten Anordnung zu verbinden, wird der Schaft 2a des chirurgischen Instruments 2 von seinem distalen Ende her durch die Durchgangsöffnung 12 geschoben, bis das Kupplungselement 13 des zweiten Adapters 6 an der Handhabe 2b des chirurgischen Instruments 2 anstößt. Dort liegen am Instrument 2 weitere Verbindungselemente vor, wie auch **Fig. 10** zeigt, nämlich ein korrespondierendes Kupplungselement 30, das korrespondierend zu der geformten Ausnehmung 29 auskragt, sodass sie ineinandergreifen, wenn beim Kuppeln das Kupplungselement 13 quasi übergestülpt wird. Der Adapter 6 kann nicht über die Handhabe 2b hinweg rutschen. Ferner wird die Ausrichtung des zweiten Adapters 6 am chirurgischen Instrument 2 durch die Nut 31 an der Auskragung 30 eindeutig festgelegt, in die der Ausrichtungsstift 28 beim Überstülpen eingeführt werden muss. Die Ausrichtung kann auch durch die geformte Ausnehmung 29 und die Auskragung 30 allein bereits bestimmt und der Ausrichtungsstift 28 nicht nötig sein. Bevorzugt sind die geformte Ausnehmung 29 und die Auskragung 30 zueinander um 180 ° rotationssymmetrisch, die Kombination aus Ausrichtungsstift 28 und Nut 31 bestimmt die Ausrichtung, wie auch **Fig. 10** in Verbindung mit **Fig. 8** zeigt. Entkuppeln erfolgt analog durch Zurückschieben des Rohrstücks 6b über den Schaft 2a.

Alternativ ist es denkbar, dass der zweite Adapter seitlich über den Schaft gesteckt, gebogen oder anderweitig verbunden wird, etwa mit Klett- oder Klebeband. Eine andere Position des zweiten Adapters am Schaft oder auch teilweise über die Handhabe geschoben ist alternativ möglich. Zu beachten ist dabei nur, dass danach das korrespondierende Eingriffselement des zweiten Adapters so vorliegt, dass es mit dem zweiten Eingriffselement in der vorgesehenen Orientierung verbunden werden kann und damit auch die Richtung des Schafts vorgibt.

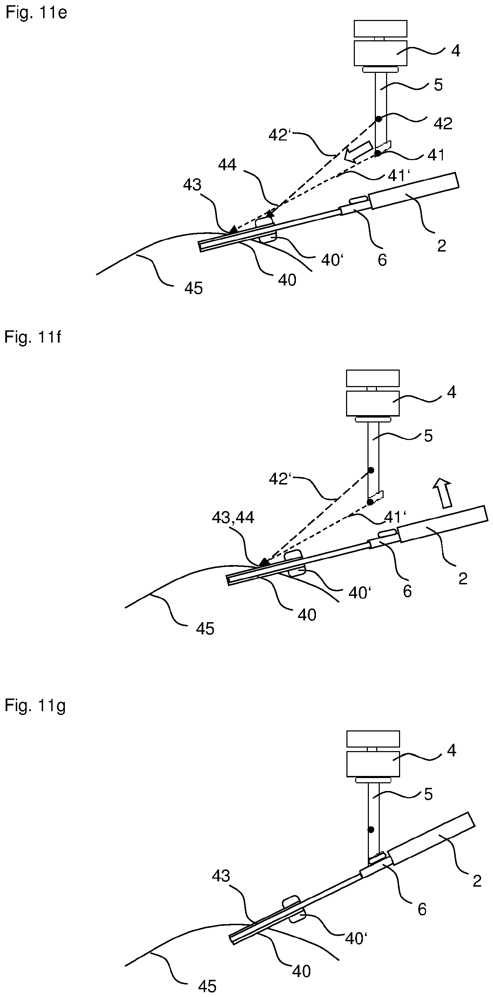

**Fig. 11****a** bis **11g** zeigen relevante Schritte des erfindungsgemäßen Verfahrens zum Verbinden des chirurgischen Instruments 2 mit dem Haltearm, wobei sowohl der Haltearm als auch das Instrument 2 so vorpositioniert werden, dass ein belastungsarmes Verbinden sowie eine sinnvolle Ausgangslage für einen folgenden chirurgischen Eingriff möglich werden. Das Vorpositionieren kann über die Steuerungsvorrichtung des Haltearms unterstützt werden, um das Verfahren zum Verbinden des chirurgischen Instruments 2 mit dem Haltearm noch weiter zu erleichtern.

Bewegungen werden jeweils in Richtung des Blockpfeils durchgeführt (auch in eine dritte Dimension außerhalb der Zeichenebene), wobei in a) und b) das chirurgische Instrument noch nicht in einem Pivotpunkt 43 gegenüber einem Körper 45 fixiert ist, also haben die Bewegungen keinen Einfluss auf den Körper 45 und sind somit nahezu frei ausführbar. Ab der in **Fig. 11c** dargestellten Andocksituation darf das chirurgische Instrument 2 nur noch um einen fixen Pivotpunkt 43 herum gegenüber dem Körper 45 bewegt werden, daher werden in c) bis e) der Haltearm und der erste Adapter 5 bewegt, und nur in f) bis g) das chirurgische Instrument 2 und der zweite Adapter 6.

In **Fig. 11a** wird der erste Adapter 5 über das Flanschstück 4 mit dem Haltearm verbunden. Dazu wird der Haltekonus 18 in die Ausnehmung 23 des Flanschstücks 4 eingeführt und durch Eingreifen der Halteschiene 24 in die Ringnut 22 des Haltekonus 18 gesichert. Alternativ kann ein zweites Eingriffselement des ersten Adapters 5 direkt mit einem korrespondierenden Eingriffselement des Haltearms, also ohne Flanschstück 4, verbunden werden.

Fig. **11b** zeigt das Anordnen des zweiten Adapters 6 an dem chirurgischen Instrument 2, was über Einführen des Schafts 2a des chirurgischen Instruments 2 in die Durchgangsöffnung 12 des Rohrstücks 6b des zweiten Adapters 6 und Ankoppeln des Kupplungselements 13 an das korrespondierende Kupplungselement 30 am proximalen Ende des Schafts 2a erfolgen kann.

In **Fig. 11c** ist das chirurgische Instrument 2 in einem Trokar 40 platziert, der in einem Körper 45 gelagert ist. Dabei bildet die Einstichstelle des Trokars 40 in der Oberfläche des Körpers 45 (bspw. bei einem laparoskopischen Eingriff die Bauchdecke des Patienten) einen Pivotpunkt 43 gegenüber dem Körper 45, um den herum das Instrument 2 (und der Trokar 40) geschwenkt und entlang seiner Längsachse verschoben und rotiert werden darf, ohne dass der Körper 45 Schaden nimmt. Der Pivotpunkt ist aber insofern fix als eine Verlagerung desselben die Einstichstelle unnötig weitet und verletzt.

Es ist nun bereits möglich, die beiden Adapter 5, 6 zueinander zu führen und miteinander zu verbinden, sodass insgesamt das chirurgische Instrument 2 mit dem Haltearm verbunden und von diesem gehalten wird. Durch die aufeinander abgestimmten Eingriffselemente der beiden Adapter 5, 6 ist dieser letzte Verbindungsschritt besonders einfach und verlässlich durchzuführen.

Besonders vorteilhaft kann jedoch der Haltearm so vorpositioniert werden, dass der erste Adapter 5 in eine für den letzten Verbindungsschritt ideale Lage gebracht wird und zugleich der Ort des Pivotpunkts 43 in Bezug auf den Haltearm bekannt ist. Dazu wird eine erste Laservorrichtung 41, die an der ersten Öffnung 3 des ersten Adapters 5 bereits angeordnet ist oder nun eingesetzt wird, aktiviert, und der entlang der durch die erste Öffnung 3 vorgegebenen Richtung ausgesendete erste Laserstrahl 41' wird auf der Oberfläche des Körpers 45 sichtbar. Haltearm und erster Adapter 5 werden dann so im Raum positioniert, dass der erste Laserstrahl 41' durch den Pivotpunkt 43 verläuft, wie **Fig. 11d** zeigt. Dazu kann es notwendig sein, das chirurgische Instrument 2 und den Trokar 40 leicht zu verkippen, sodass der Trokarkopf 40' den Verlauf des ersten Laserstrahls 41' nicht blockiert.

Anschließend wird eine zweite Laservorrichtung 42, die an der zweiten Öffnung 7 des ersten Adapters 5 bereits angeordnet ist oder nun eingesetzt wird, aktiviert, wie in **Fig. 11e** dargestellt. Der in einer vorbestimmten Richtung ausgesendete zweite Laserstrahl 42' schneidet den ersten Laserstrahl 41' in einem durch die Winkel α, β der Öffnungen 3, 7 gegenüber der Längsachse Z des länglichen Abschnitts 5c und durch den Abstand der Öffnungen 3, 7 zueinander vorbestimmten Schnittpunkt 44. Um diesen Schnittpunkt 44 auf den Pivotpunkt 43 zu legen, werden Haltearm und erster Adapter 5 entlang der Richtung des ersten Laserstrahls 41', also parallel zu diesem, bewegt, bis auch der zweite Laserstrahl 42' durch den Pivotpunkt 43 verläuft, der folglich nun im Schnittpunkt 44 der Laserstrahlen 41', 42' liegt, wie **Fig. 11f** zeigt.

Nun können die Laservorrichtungen 41, 42 wieder deaktiviert werden und die beiden Adapter 5,6 können aneinander gekoppelt werden, indem das chirurgische Instrument 2 mit dem zweiten Adapter 6 zu der Position des ersten Adapters 5 am Haltearm geführt wird, dargestellt in **Fig. 11g**, wobei das chirurgische Instrument 2 um den fixen Pivotpunkt 43 geschwenkt wird. Die Adapter 5, 6 werden in Eingriff gebracht, indem das chirurgische Instrument 2 entlang seiner Längsachse verschoben wird und der Verbindungskörper 8 in den Aufnahmeraum 11a des Schienenelements 11 eingefädelt wird. Die Verbindung kann gesichert werden, indem die Nase 10a des Sicherungsclips 10 in der Einkerbung 9 des Verbindungskörpers 8 aufgenommen wird.

Der Pivotpunkt 43 liegt damit an einer für das System bekannten Stelle, ohne dass er an gezielt an dieser Stelle gelegt werden musste.

Die chirurgische Haltevorrichtung, an deren zumindest einen Haltearm das chirurgische Instrument 2 angedockt wird, kann Teil eines steuerbaren Chirurgie-Roboters sein, in dessen Steuerungsvorrichtung ein Andockprogramm hinterlegt sein kann, sodass die beschriebenen Schritte durch die Haltevorrichtung gestützt ablaufen können. Auf bestimmte Nutzereingaben hin, bspw. Betätigen eines Tasters oder Eingeben von Softwarebefehlen, werden dann die Laservorrichtungen 41, 42 aktiviert bzw. deaktiviert oder es werden Bewegungen des mit dem ersten Adapter 5 verbundenen Haltearms gesperrt, sodass, wenn die in **Fig. 11d** / **11e** dargestellte Zwischenposition erreicht ist, der Haltearm nur noch entlang einer zum ersten Lasterstrahl 41' parallelen Achse verschoben werden kann. Dadurch wird sichergestellt, dass die zuvor eingestellte Position, in der der erste Lasterstrahl 41' durch den Pivotpunkt 43 verläuft, nicht mehr verlassen wird.

Auch kann die korrekte Positionierung des zweiten Laserstrahls 42' und damit die finale Andocksituation bestätigt werden, sodass die Steuerungsvorrichtung den Ort des Pivotpunkts 43 durch den Schnittpunkt 44 der Laserstrahlen 41', 42' kennt und hinterlegt, damit weitere Bewegungen, die das chirurgische Instrument 2 durchführen soll, so durch Bewegungen des Haltearms übertragen werden, dass dabei der Pivotpunkt 43 nicht verlagert wird.

Nach der Verwendung des chirurgischen Instruments können die mit der erfindungsgemäßen Andockvorrichtung 1 herbeigeführten Verbindungen wieder gelöst werden. Das Flanschstück, sofern eines verwendet wurde, kann am Gegenflansch verbleiben. Die zweiteilige Adaptervorrichtung kann entsorgt und ersetzt oder sterilisiert und für eine weitere Verwendung vorbereitet werden.

### BEZUGSZEICHENLISTE

- 1: Andockvorrichtung
- 2: chirurgisches Instrument
- 2a: Schaft
- 2b: Handhabe
- 3: erste Öffnung
- 4: Flanschstück
- 4': Flanschkörper
- 4": Hülse
- 5: erster Adapter
- 5a: proximales Ende erster Adapter
- 5b: distales Ende erster Adapter
- 5c: länglicher Abschnitt erster Adapter
- 6: zweiter Adapter
- 6a: Schienenabschnitt zweiter Adapter
- 6b: Rohrstück zweiter Adapter
- 7: zweite Öffnung
- 8: Verbindungskörper
- 8': endständige Verbindungsfläche Endabschnitt
- 8": zum länglichen Abschnitt weisende Seite Endabschnitt
- 9: Einkerbung
- 10: Sicherungsclip
- 10a: Nase
- 11: Schienenelement
- 11a: Aufnahmeraum Schienenelement
- 11': Seitenwand Schienenelement
- 11": Rückwand Schienenelement
- 12: Durchgangsöffnung
- 13: Kupplungselement, Verbindungsring
- 14: Greifnippel
- 15: Sicherungsauskragung
- 16: Gegenflansch
- 17: Verschraubung
- 18: Haltekonus
- 18': Spitze Haltekonus (Pin zum Durchstoßen)
- 20: Ringnut
- 21: Kragen
- 22: Ausrichtungsstift erster Adapter
- 23: Ausnehmung
- 24: Halteschiene
- 25: Führung der Halteschiene
- 26: Feder
- 27: Stift
- 28: Ausrichtungsstift zweiter Adapter
- 29: geformte Ausnehmung
- 30: Auskragung
- 31: Nut
- 40: Trokar
- 40': Trokarkopf
- 41: erste Laservorrichtung
- 41': erster Laserstrahl
- 42: zweite Laservorrichtung
- 42': zweiter Laserstrahl
- 43: Pivotpunkt
- 44: Schnittpunkt Laserstrahlen
- 45: Körper

- Z: Längsachse

- α: erster Winkel
- β: zweiter Winkel

## Patentansprüche

1. Andockvorrichtung (1), die dazu ausgebildet ist, ein chirurgisches Instrument (2) mit einem Haltearm einer chirurgischen Haltevorrichtung in einer Andocksituation zu verbinden, wobei das chirurgische Instrument (2) einen stabförmigen Schaft (2a) und eine Handhabe (2b) aufweist und dazu ausgebildet ist, mittels einem Trokar (40) geführt zu werden,
wobei die Andockvorrichtung (1) eine zweiteilige Adaptervorrichtung aufweist, die an einem proximalen Ende mit dem Haltearm und an einem distalen Ende mit dem chirurgischen Instrument (2) verbindbar ist, wobei
die zweiteilige Adaptervorrichtung
- einen ersten Adapter (5)
mit einem länglichen Abschnitt (5c), der eine Längsachse (Z) aufweist, und
mit einem Verbindungskörper (8), der an einem distalen Ende (5b) des ersten Adapters (5) vorliegt, und der ein erstes Eingriffselement mit einer endständigen Verbindungsfläche (8') aufweist, und
- einen zweiten Adapter (6),
der dazu ausgebildet ist, an dem Schaft (2a) des chirurgischen Instruments (2) angeordnet zu werden und der ein in der Andocksituation zu der endständigen Verbindungsfläche (8') weisendes und mit dieser korrespondierendes Eingriffselement (11) hat,
aufweist,
wobei der Verbindungskörper (8) des ersten Adapters (5) mit dem korrespondierenden Eingriffselement (11) des zweiten Adapters (6) in der Andocksituation eine lösbare Verbindung bildet,
**dadurch gekennzeichnet, dass**
die endständige Verbindungsfläche (8') des ersten Adapters (5) in Bezug auf dessen Längsachse (Z) um einen vorbestimmten ersten Winkel (α), der in einem Bereich von 45° bis 75° liegt, geneigt ist.

2. Andockvorrichtung (1) nach Anspruch 1,
**dadurch gekennzeichnet, dass**
der Verbindungskörper (8) des ersten Adapters (5) zumindest mit seinem die Verbindungsfläche (8') aufweisenden Abschnitt als ein Schlitten (8) ausgebildet ist und das korrespondierende Eingriffselement (11) des zweiten Adapters (6) als ein Schienenelement (11) zur Aufnahme des Schlittens (8) ausgebildet ist.

3. Andockvorrichtung (1) nach Anspruch 1 oder 2,
**dadurch gekennzeichnet, dass**
der Verbindungskörper (8) eine Aufnahme für eine erste Laservorrichtung (41) bildet und eine erste Öffnung (3) als Austrittsöffnung für einen ersten Laserstrahl (41') aufweist,
und der längliche Abschnitt (5c) des ersten Adapters (5) eine Aufnahme für eine zweite Laservorrichtung (42) bildet und eine zweite Öffnung (7) als Austrittsöffnung für einen zweiten Laserstrahl (42') aufweist, wobei der Laserstrahl (42') in einem zweiten Winkel (β) gegenüber der Längsachse (Z) des ersten Adapters (5) verläuft, der kleiner ist als der erste Winkel (α) und in einem Bereich von 20 ° bis 80 ° liegt und wobei der aus der Öffnung (3) austretende erste Laserstrahl (41') den aus der zweiten Öffnung (7) austretenden zweiten Laserstrahl (42') in einem vorbestimmten Schnittpunkt (44) schneidet.

4. Andockvorrichtung (1) nach Anspruch 3,
**dadurch gekennzeichnet, dass**
die erste Öffnung (3) ein Loch in der Wandung des Verbindungskörpers (8), eine Durchgangsöffnung (3) oder eine zylindrische Ausnehmung in dem Verbindungskörper (8) ist, wobei bevorzugt die erste Öffnung (3) winklig angefaste Ränder aufweist, und/oder
die zweite Öffnung (7) ein Loch in der Wandung des ersten Adapters (5), eine Durchgangsöffnung (7) oder eine zylindrische Ausnehmung in dem ersten Adapter (5) ist, wobei bevorzugt die zweite Öffnung (7) winklig angefaste Ränder aufweist, und/oder wobei bevorzugt die erste Öffnung (3) an die endständige Verbindungsfläche (8') angrenzt.

5. Andockvorrichtung (1) nach zumindest einem der Ansprüche 1 bis 4,
**dadurch gekennzeichnet, dass**
das korrespondierende Eingriffselement (11) des zweiten Adapters (6) über einem Rohrstück (6b) mit einer zylindrischen Durchtrittsöffnung (12) des zweiten Adapters (6) angeordnet ist, durch die der Schaft (2a) des chirurgischen Instruments (2) führbar ist.

6. Andockvorrichtung (1) nach Anspruch 5,
**dadurch gekennzeichnet, dass**
das Rohrstück (6b) ein Kupplungselement (13) aufweist, das mit einem korrespondierenden Kupplungselement (30) am proximalen Ende des Schafts (2a) des chirurgischen Instruments (2) kuppelbar ist.

7. Andockvorrichtung (1) nach zumindest einem der Ansprüche 1 bis 6,
**dadurch gekennzeichnet, dass**
der erste Adapter (5) an seinem proximalen Ende (5a) ein zweites Eingriffselement aufweist, das dazu ausgebildet ist, mit einem korrespondierenden Eingriffselement des Haltearms lösbar verbunden zu werden.

8. Andockvorrichtung (1) nach zumindest einem der Ansprüche 1 bis 7,
**dadurch gekennzeichnet, dass**
der erste Adapter (5) an seinem proximalen Ende einen Haltekonus (18) aufweist, der sich zu einer proximalen Spitze (18') hin verjüngt.

9. Andockvorrichtung (1) nach zumindest einem der Ansprüche 1 bis 8,
**dadurch gekennzeichnet, dass**
die Andockvorrichtung (1) ein Flanschstück (4) aufweist, das dazu ausgebildet ist, an einem korrespondierenden Gegenflansch (16) des Haltearms angeordnet zu werden, wobei bevorzugt das Flanschstück (4) eine konusförmige Ausnehmung (23) aufweist, die zur Aufnahme des Haltekonus (18) mit einer äußeren Umfangsform des Haltekonus (18) korrespondiert.

10. Chirurgische Haltevorrichtung, die zumindest einen Haltearm aufweist, der über eine Andockvorrichtung in einer Andocksituation mit einem chirurgischen Instrument (2) verbunden ist, das einen stabförmigen Schaft (2a) und eine Handhabe (2b) aufweist und dazu ausgebildet ist, durch einen Trokar (40) geführt zu werden,
**dadurch gekennzeichnet, dass**
die Andockvorrichtung eine Andockvorrichtung (1) nach zumindest einem der Ansprüche 1 bis 9 ist.

11. Chirurgische Haltevorrichtung nach Anspruch 10,
**dadurch gekennzeichnet, dass**
die chirurgische Haltevorrichtung ein Chirurgie-Roboter mit zumindest einem Roboterkopf ist, wobei der Chirurgie-Roboter eine Steuerungsvorrichtung zum gesteuerten Bewegen des Haltearms aufweist.

12. Chirurgische Haltevorrichtung nach Anspruch 11,
**dadurch gekennzeichnet, dass**
die Steuerungsvorrichtung mit einer ersten Laservorrichtung (41), die bevorzugt an der ersten Öffnung (3), und einer zweiten Laservorrichtung (42), die bevorzugt an der zweiten Öffnung (7) des ersten Adapters (5) der Andockvorrichtung (1) angeordnet ist, verbindbar ist, wobei die Laservorrichtungen (41, 42) so zueinander ausgerichtet sind, dass sich deren Laserstrahlen (41', 42') in einem vorbestimmten Schnittpunkt (44) schneiden,
wobei die Laservorrichtungen (41, 42) Laserfasern, die mit einer Laserstrahlquelle verbindbar sind, oder Lasermodule mit eigener Laserstrahlquelle sind.

## Claims

1. A docking device (1) which is designed to connect a surgical instrument (2) to a holding arm of a surgical holding device in a docking situation, wherein the surgical instrument (2) has a rod-shaped shaft (2a) and a handle (2b) and is designed to be guided by means of a trocar (40),
wherein
the docking device (1) has a two-part adapter device which can be connected at a proximal end to the holding arm and at a distal end to the surgical instrument (2), wherein
the two-part adapter device has
- a first adapter (5)
with an elongated section (5c) having a longitudinal axis (Z), and a connecting body (8) which is present at a distal end (5b) of the first adapter (5) and which has a first engagement element with a terminal connection surface (8'), and
- a second adapter (6),
which is designed to be arranged on the shaft (2a) of the surgical instrument (2) and which has an engagement element (11) pointing towards and corresponding to the terminal connection surface (8') in the docking situation,
wherein the connecting body (8) of the first adapter (5) forms a detachable connection with the corresponding engagement element (11) of the second adapter (6) in the docking situation,
**characterised in that**
the terminal connection surface (8') of the first adapter (5) is inclined in relation to its longitudinal axis (Z) by a predetermined first angle (α), which is in a range from 45° to 75°.

2. The docking device (1) according to claim 1,
**characterised in that**
the connecting body (8) of the first adapter (5) is designed as a slide (8), at least with its section having the connection surface (8'), and the corresponding engagement element (11) of the second adapter (6) is designed as a rail element (11) for receiving the slide (8).

3. The docking device (1) according to claim 1 or 2,
**characterised in that**
the connecting body (8) forms a receptacle for a first laser device (41) and has a first opening (3) as an exit opening for a first laser beam (41'),
and the elongated section (5c) of the first adapter (5) forms a receptacle for a second laser device (42) and has a second opening (7) as an exit opening for a second laser beam (42'), wherein the laser beam (42') runs at a second angle (β) with respect to the longitudinal axis (Z) of the first adapter (5), which is smaller than the first angle (α) and lies in a range from 20° to 80° and wherein the first laser beam (41') emerging from the opening (3) intersects the second laser beam (42') emerging from the second opening (7) at a predetermined intersection point (44).

4. The docking device (1) according to claim 3, **characterised in that**
the first opening (3) is a hole in the wall of the connecting body (8), a through-opening (3) or a cylindrical recess in the connecting body (8), wherein the first opening (3) preferably has edges bevelled at an angle, and/or
the second opening (7) is a hole in the wall of the first adapter (5), a through-opening (7) or a cylindrical recess in the first adapter (5), wherein the second opening (7) preferably has edges bevelled at an angle, and/or wherein the first opening (3) preferably adjoins the terminal connection surface (8').

5. The docking device (1) according to at least one of claims 1 to 4,
**characterised in that**
the corresponding engagement element (11) of the second adapter (6) is arranged over a tubular piece (6b) with a cylindrical passage opening (12) of the second adapter (6), through which the shaft (2a) of the surgical instrument (2) can be guided.

6. The docking device (1) according to claim 5,
**characterised in that**
the tubular piece (6b) has a coupling element (13) which can be coupled to a corresponding coupling element (30) at the proximal end of the shaft (2a) of the surgical instrument (2).

7. The docking device (1) according to at least one of claims 1 to 6,
**characterised in that**
the first adapter (5) has, at its proximal end (5a), a second engagement element, which is designed to be detachably connected to a corresponding engagement element of the holding arm.

8. The docking device (1) according to at least one of claims 1 to 7,
**characterised in that**
the first adapter (5) has, at its proximal end, a holding cone (18), which tapers towards a proximal tip (18').

9. The docking device (1) according to at least one of claims 1 to 8,
**characterised in that**
the docking device (1) has a flange piece (4) which is designed to be arranged on a corresponding mating flange (16) of the holding arm, wherein the flange piece (4) preferably has a conical recess (23) which corresponds to an outer circumferential shape of the holding cone (18) for receiving the holding cone (18).

10. A surgical holding device which has at least one holding arm which is connected via a docking device in a docking situation to a surgical instrument (2) which has a rod-shaped shaft (2a) and a handle (2b) and is designed to be guided through a trocar (40),
**characterised in that**
the docking device is a docking device (1) according to at least one of claims 1 to 9.

11. The surgical holding device according to claim 10,
**characterised in that**
the surgical holding device is a surgical robot with at least one robot head, wherein the surgical robot has a control device for the controlled movement of the holding arm.

12. The surgical holding device according to claim 11,
**characterised in that**
the control device can be connected to a first laser device (41), which is preferably arranged at the first opening (3), and a second laser device (42), which is preferably arranged at the second opening (7) of the first adapter (5) of the docking device (1), wherein the laser devices (41, 42) are aligned with respect to one another such that their laser beams (41', 42') intersect at a predetermined intersection point (44),
wherein the laser devices (41, 42) are laser fibres which can be connected to a laser beam source, or laser modules with their own laser beam source.

## Revendications

1. Dispositif d'amarrage (1) conçu pour connecter un instrument chirurgical (2) à un bras de maintien d'un dispositif de maintien chirurgical dans une situation d'amarrage, dans lequel l'instrument chirurgical (2) comporte un arbre (2a) en forme de tige et une poignée (2b) et est conçu pour être guidé au moyen d'un trocart (40),
dans lequel
le dispositif d'amarrage (1) comporte un dispositif adaptateur en deux parties qui peut être connecté à une extrémité proximale au bras de maintien et à une extrémité distale à l'instrument chirurgical (2), dans lequel
le dispositif adaptateur en deux parties a
- un premier adaptateur (5)
avec une section allongée (5c) ayant un axe longitudinal (Z), et un corps de connexion (8) qui est présent à une extrémité distale (5b) du premier adaptateur (5) et qui a un premier élément d'engagement avec une surface de connexion terminale (8'), et
- un deuxième adaptateur (6),
qui est conçu pour être disposé sur l'arbre (2a) de l'instrument chirurgical (2) et qui a un élément d'engagement (11) pointant vers et correspondant à la surface de connexion terminale (8') dans la situation d'amarrage,
dans lequel le corps de connexion (8) du premier adaptateur (5) forme une connexion détachable avec l'élément d'engagement correspondant (11) du deuxième adaptateur (6) dans la situation d'amarrage,
**caractérisé en ce que**
la surface de connexion terminale (8') du premier adaptateur (5) est inclinée par rapport à son axe longitudinal (Z) d'un premier angle prédéterminé (α), qui se situe dans une plage allant de 45° à 75°.

2. Dispositif d'amarrage (1) selon la revendication 1,
**caractérisé en ce que**
le corps de connexion (8) du premier adaptateur (5) est conçu comme un coulisseau (8), au moins avec sa section comportant la surface de connexion (8'), et l'élément d'engagement (11) correspondant du deuxième adaptateur (6) est conçu comme un élément de rail (11) pour recevoir le coulisseau (8).

3. Dispositif d'amarrage (1) selon la revendication 1 ou 2,
**caractérisé en ce que**
le corps de connexion (8) forme un réceptacle pour un premier dispositif laser (41) et a un premier orifice (3) comme orifice de sortie pour un premier faisceau laser (41'),
et la section allongée (5c) du premier adaptateur (5) forme un réceptacle pour un deuxième dispositif laser (42) et a un deuxième orifice (7) comme orifice de sortie pour un deuxième faisceau laser (42'), dans lequel le faisceau laser (42') s'étend selon un deuxième angle (β) par rapport à l'axe longitudinal (Z) du premier adaptateur (5), qui est plus petit que le premier angle (α) et se situe dans une plage de 20° à 80° et dans lequel le premier faisceau laser (41') sortant de l'orifice (3) croise le deuxième faisceau laser (42') sortant du deuxième orifice (7) en un point de croisement prédéterminé (44).

4. Dispositif d'amarrage (1) selon la revendication 3,
**caractérisé en ce que**
le premier orifice (3) est un trou dans la paroi du corps de connexion (8), un orifice de passage (3) ou un évidement cylindrique dans le corps de connexion (8), dans lequel le premier orifice (3) a de préférence des bords biseautés à un angle, et/ou le deuxième orifice (7) est un trou dans la paroi du premier adaptateur (5), un orifice de passage (7) ou un évidement cylindrique dans le premier adaptateur (5), dans lequel le deuxième orifice (7) a de préférence des bords biseautés à un angle, et/ou dans lequel le premier orifice (3) jouxte de préférence la surface de connexion terminale (8').

5. Dispositif d'amarrage (1) selon au moins l'une des revendications 1 à 4, **caractérisé en ce que**
l'élément d'engagement correspondant (11) du deuxième adaptateur (6) est disposé sur une pièce tubulaire (6b) avec un orifice de passage cylindrique (12) du deuxième adaptateur (6), à travers lequel l'arbre (2a) de l'instrument chirurgical (2) peut être guidé.

6. Dispositif d'amarrage (1) selon la revendication 5,
**caractérisé en ce que**
la pièce tubulaire (6b) comporte un élément d'accouplement (13) qui peut être accouplé à un élément d'accouplement correspondant (30) à l'extrémité proximale de l'arbre (2a) de l'instrument chirurgical (2).

7. Dispositif d'amarrage (1) selon au moins l'une des revendications 1 à 6, **caractérisé en ce que**
le premier adaptateur (5) a, à son extrémité proximale (5a), un deuxième élément d'engagement, qui est conçu pour être connecté de manière détachable à un élément d'engagement correspondant du bras de maintien.

8. Dispositif d'amarrage (1) selon au moins l'une des revendications 1 à 7, **caractérisé en ce que**
le premier adaptateur (5) a, à son extrémité proximale, un cône de maintien (18), qui se rétrécit vers une pointe proximale (18').

9. Dispositif d'amarrage (1) selon au moins l'une des revendications 1 à 8, **caractérisé en ce que**
le dispositif d'amarrage (1) a une pièce de bride (4) qui est conçue pour être disposée sur une bride de raccordement correspondante (16) du bras de maintien, dans lequel la pièce de bride (4) a de préférence un évidement conique (23) qui correspond à une forme circonférentielle extérieure du cône de maintien (18) pour recevoir le cône de maintien (18).

10. Dispositif de maintien chirurgical qui a au moins un bras de maintien qui est connecté par l'intermédiaire d'un dispositif d'amarrage dans une situation d'amarrage à un instrument chirurgical (2) qui comporte un arbre (2a) en forme de tige et une poignée (2b) et est conçu pour être guidé à travers un trocart (40), **caractérisé en ce que**
le dispositif d'amarrage est un dispositif d'amarrage (1) selon au moins l'une des revendications 1 à 9.

11. Dispositif de maintien chirurgical selon la revendication 10,
**caractérisé en ce que**
le dispositif de maintien chirurgical est un robot chirurgical comportant au moins une tête de robot, dans lequel le robot chirurgical a un dispositif de contrôle pour le mouvement contrôlé du bras de maintien.

12. Dispositif de maintien chirurgical selon la revendication 11,
**caractérisé en ce que**
le dispositif de contrôle peut être connecté à un premier dispositif laser (41), qui est de préférence disposé au niveau du premier orifice (3), et un deuxième dispositif laser (42), qui est disposé de préférence au niveau du deuxième orifice (7) du premier adaptateur (5) du dispositif d'amarrage (1), dans lequel les dispositifs laser (41, 42) sont alignés les uns par rapport aux autres de telle sorte que leurs faisceaux laser (41', 42') se croisent en un point de croisement prédéterminé (44),
dans lequel les dispositifs laser (41, 42) sont des fibres laser qui peuvent être connectées à une source de faisceau laser, ou des modules laser avec leur propre source de faisceau laser.
